(19) **Europäisches Patentamt**
**European Patent Office**
**Office européen des brevets**

(11) **EP 3 491 945 B1**

(12) **EUROPEAN PATENT SPECIFICATION**

(45) Date of publication and mention
of the grant of the patent:
**08.12.2021 Bulletin 2021/49**

(51) Int Cl.:
*A24F 40/30* *(2020.01)* *A24F 40/50* *(2020.01)*
*A24F 40/51* *(2020.01)* *A24F 40/485* *(2020.01)*

(21) Application number: **16910502.0**

(86) International application number:
**PCT/JP2016/071998**

(22) Date of filing: **27.07.2016**

(87) International publication number:
**WO 2018/020599 (01.02.2018 Gazette 2018/05)**

(54) **FLAVOR INHALER**

AROMAINHALATOR

INHALATEUR D'ARÔME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.06.2019 Bulletin 2019/23**

(73) Proprietor: **Japan Tobacco Inc.**
**Tokyo 105-8422 (JP)**

(72) Inventors:
• **NAKANO, Takuma**
**Tokyo 130-8603 (JP)**

• **SUZUKI, Akihiko**
**Tokyo 130-8603 (JP)**
• **YAMADA, Manabu**
**Tokyo 130-8603 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 617 303        EP-A1- 2 989 912**
**EP-A2- 3 261 468        WO-A1-2014/110119**
**WO-A1-2014/115324      US-A1- 2015 258 288**

EP 3 491 945 B1

**Description**

TECHNICAL FIELD

The present invention relates to a flavor inhaler for making flavor to be included in aerosol and inhaled.

BACKGROUND ART

Since some time in the past, a type of flavor inhaler, by which flavor is inhaled without a burning process, has been known. For example, a flavor inhaler comprises an atomizing unit for atomizing an aerosol source without a burning process, and a flavor source arranged in a position closer to a mouthpiece than the position of the atomizing unit (for example, refer to WO 2015/179388 A1).

US 2015/258288 A1 is related to the preamble of claims 1 to 4.

SUMMARY OF INVENTION

TECHNICAL PROBLEM

In the flavor inhaler disclosed in WO 2015/179388 A1, the amount of flavor to be inhaled can be adjusted by changing the amount of aerosol generated in the atomizing unit. However, the degree of freedom of control of the amount of aerosol and the amount of flavor to be inhaled is not large.

The present invention has been made by taking the above matters into consideration; and an object of the present invention is to provide a flavor inhaler which can improve the degree of freedom of control of the amount of aerosol and the amount of flavor to be inhaled.

SOLUTION TO PROBLEM

For solving the above problem, a flavor inhaler according to the present invention comprises the features of one of claims 1 to 4.

Another mode of the present invention comprises the flavor inhaler of the above mode, wherein the second flow path is a flow path which does not pass through the flavor source.

Another mode of the present invention comprises the flavor inhaler of the above mode, wherein the flow rate adjustment mechanism is arranged at least in a part on the first flow path or the second flow path.

Another mode of the present invention comprises the flavor inhaler of the above mode, wherein the flavor inhaler comprises a control section for controlling operation of at least one of the flow rate adjustment mechanism and the atomizing section.

Another mode of the present invention comprises the flavor inhaler of the above mode, wherein the control section is constructed in such a manner that the control section controls the ratio between the air flow rate of the first flow path and the air flow rate of the second flow path, by controlling operation of the flow rate adjustment mechanism based on the amount of the aerosol generated in the atomizing section.

Another mode of the present invention comprises the flavor inhaler of the above mode, wherein the amount of the aerosol to be generated in the atomizing section per predetermined time, at the time when inhaling action starts, is determined in advance; and the control section controls the air flow rate ratio, based on the predetermined amount of the aerosol to be generated per the predetermined time.

Another mode of the present invention comprises the flavor inhaler of the above mode, wherein the control section is further constructed in such a manner that the control section detects change in the amount of the aerosol to be generated in the atomizing section per the predetermined time, and the control section controls the air flow rate ratio based on the changed amount of the aerosol to be generated per the predetermined time.

Another mode of the present invention comprises the flavor inhaler of the above mode, wherein the control section is constructed in such a manner that the control section controls the amount of the aerosol to be generated in the atomizing section by controlling operation of the atomizing section based on the ratio between the air flow rate of the first flow path and the air flow rate of the second flow path.

Another mode of the present invention comprises the flavor inhaler of the above mode, wherein the air flow rate ratio, at the time when inhaling action starts, is determined in advance; and the control section controls the amount of the aerosol to be generated in the atomizing section based on the predetermined air flow rate ratio.

Another mode of the present invention comprises the flavor inhaler of the above mode, wherein the control section is further constructed in such a manner that the control section detects change in the air flow rate ratio, and the control section controls the amount of the aerosol to be generated in the atomizing section based on the changed air flow rate ratio.

Another mode of the present invention comprises the flavor inhaler of the above mode, wherein the control section determines the changed air flow rate ratio based on an operation state of the flow rate adjustment mechanism.

Another mode of the present invention comprises the flavor inhaler of the above mode, wherein the control section controls at least one of the air flow rate ratio and the amount of the aerosol to be generated in the atomizing section, for making the amount of the aerosol passing through the first flow path to be constant.

Another mode of the present invention comprises the flavor inhaler of the above mode, wherein the control section controls the atomizing section to change the predetermined amount of the aerosol to be generated in the atomizing section per the predetermined time, in the case that an accumulated value of the amounts of the aerosol generated in the atomizing section or an accumulated value of the amounts of the aerosol passed through the first flow path exceeds a first threshold value.

Another mode of the present invention comprises the flavor inhaler of the above mode, wherein the control section controls the flow rate adjustment mechanism to change the predetermined air flow rate ratio, in the case that an accumulated value of the amounts of the aerosol generated in the atomizing section or an accumulated value of the amounts of the aerosol passed through the first flow path exceeds a first threshold value.

Another mode of the present invention comprises the flavor inhaler of the above mode, wherein the control section is further constructed in such a manner that the control section blocks communication between the atomizing section and the first flow path or cuts off supply of electric power to the atomizing section, in the case that an accumulated value of the amounts of the aerosol generated in the atomizing section or an accumulated value of the amounts of the aerosol passed through the first flow path exceeds a second threshold value.

Another mode of the present invention comprises the flavor inhaler of the above mode, wherein the control section calculates the amount of the aerosol passed through the first flow path based on the air flow rate ratio and the amount of the aerosol generated in the atomizing section.

Another mode of the present invention comprises the flavor inhaler of the above mode, wherein the control section calculates the amount of the aerosol generated in the atomizing section based on electric energy supplied to the atomizing section.

Another mode of the present invention comprises the flavor inhaler of the above mode, wherein the flow rate adjustment mechanism comprises a mechanism for changing at least one of a cross-section area of at least a part of the first flow path and a cross-section area of at least a part of the second flow path.

Another mode of the present invention comprises the flavor inhaler of the above mode, wherein the flow rate adjustment mechanism comprises a first member and a second member; at least one of the first flow path and the second flow path is formed by the first member and the second member; and at least one of a cross-section area of at least a part of the first flow path and a cross-section area of at least a part of the second flow path is changed as a result of relative movement of the first member and the second member.

Another mode of the present invention comprises the flavor inhaler of the above mode, wherein the flavor inhaler further comprises a battery assembly comprising a battery.

Another mode of the present invention comprises the flavor inhaler of the above mode, wherein the battery assembly is attachable/detachable to/from the atomizing section.

Another mode of the present invention comprises the flavor inhaler of the above mode, wherein the flow rate adjustment mechanism is electrically connected to the battery.

Another mode of the present invention comprises the flavor inhaler of the above mode, wherein the flavor inhaler further comprises a user setting section for allowing setting of at least one of the air flow rate ratio and the amount of the aerosol to be generated in the atomizing section.

Another mode of the present invention comprises the flavor inhaler of the above mode, wherein the flavor inhaler further comprises an inhaling sensor for detecting inhaling action.

Another mode of the present invention comprises the flavor inhaler of the above mode, wherein the flow rate adjustment mechanism comprises a flow rate sensor for detecting an air flow rate of at least one of the first flow path and the second flow path.

ADVANTAGEOUS EFFECTS OF INVENTION

According to the present invention, the degree of freedom of control of the amount of aerosol and the amount of flavor to be inhaled can be improved.

BRIEF DESCRIPTION OF DRAWINGS

Fig. 1 is a figure showing a construction of a flavor inhaler 100 according to an embodiment.

Fig. 2 is a flow chart showing operation of a control section 130 relating to a first mode of control.

Fig. 3 is a flow chart showing operation of a control section 130 relating to a second mode of control.

Fig. 4 is a flow chart showing operation of a control section 130 relating to a third mode of control.

Fig. 5 is a figure showing a construction of a flavor inhaler 500 according to an embodiment.

Fig. 6 is a figure showing an aerosol flow path of the flavor inhaler 500.

Fig. 7 is a figure showing a construction of a flavor inhaler 700 according to another embodiment.

Fig. 8 is a figure showing an example of a construction of a flow rate adjustment mechanism 730 and operation thereof.

Fig. 9 is a figure showing another example of a construction of a flow rate adjustment mechanism 730 and operation thereof.

DESCRIPTION OF EMBODIMENTS

In the following description, embodiments of the present invention will be explained with reference to the figures.

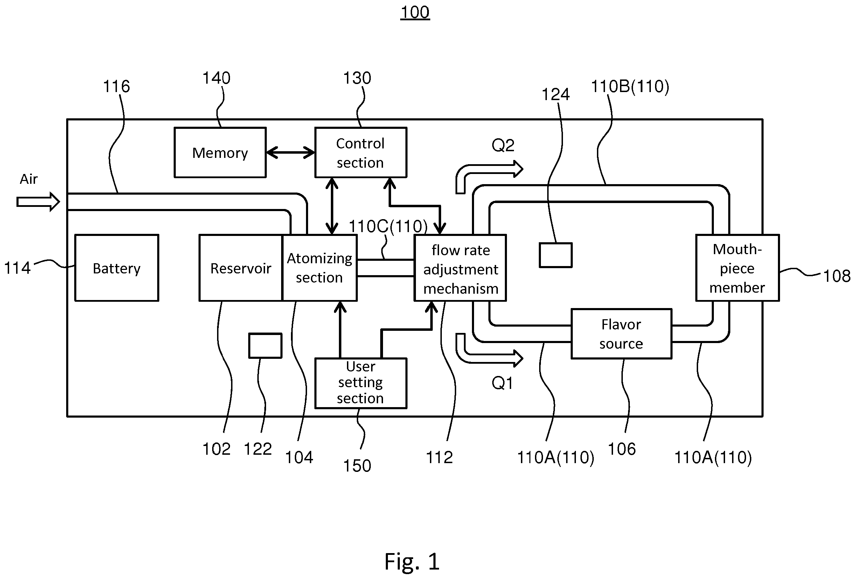

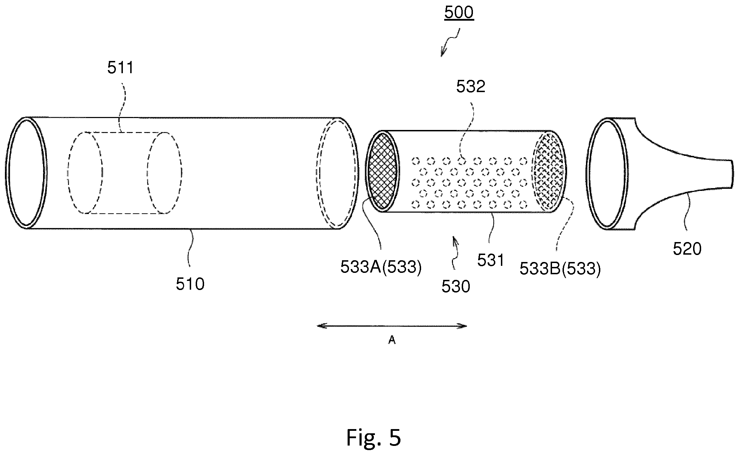

Fig. 1 is a figure showing a construction of a flavor inhaler 100 according to an embodiment of the present invention. It should be reminded that Fig. 1 shows respective elements included in the flavor inhaler 100 in a schematic and conceptual manner, and does not show precise arrangement, shapes, sizes, positional relationship, and so on of the respective elements and the flavor inhaler 100. Regarding constructions and appearances of flavor inhalers and respective elements, which are more realistic and closer to actual examples, they will be explained later with reference to Figs. 5-9.

As shown in Fig. 1, the flavor inhaler 100 comprises a reservoir 102, an atomizing section 104, a flavor source 106, a mouthpiece member 108, an aerosol flow path 110, and a flow rate adjustment mechanism 112. Regarding these elements in the flavor inhaler 100, some of them may be integrated into a detachable cartridge. For example, the flavor source 106 only may be constructed as a cartridge attachable/detachable to/from a main body of the flavor inhaler 100, the atomizing section 104 and the reservoir 102 may be constructed as a cartridge attachable/detachable to/from a battery 114, and the flavor source 106, the reservoir 102, and the atomizing section 104 may be integrated into a cartridge attachable/detachable to/from the battery 114.

The reservoir 102 holds an aerosol source. For example, the reservoir 102 comprises fibrous or porous material, and holds the aerosol source, which is in the form of fluid, by spaces between fibers or in pores in the porous material. The reservoir 102 may be constructed as a tank for storing fluid. The aerosol source may be, for example, a liquid such as glycerin or propylene glycol. The reservoir 102 comprises a construction for allowing replenishment of the aerosol source, or a construction for allowing replacement of the reservoir itself when the aerosol source is exhausted.

The atomizing section 104 is constructed to generate aerosol by atomizing the aerosol source. The atomizing section 104 generates the aerosol when inhaling action is detected by an inhaling sensor 122 (for example, a pressure sensor for detecting change in pressure in an air taking-in flow path 116 or the aerosol flow path 110, or a manipulation button which can be manipulated by a user). For example, a wick is arranged for connection between the reservoir 102 and the atomizing section 104. A part of the wick extends to the inside of the reservoir 102 and is in contact with the aerosol source. Another part of the wick extends toward the atomizing section 104. The aerosol source is sent from the reservoir 102 to the atomizing section 102 by capillary effect in the wick. The atomizing section 104 comprises, for example, a heater which is electrically connected to the battery 114. The heater is arranged to be in contact with the wick, and the aerosol source sent through the wick is heated to be atomized. Another example of the atomizing section 104 may be an ultrasonic-type atomizer which atomizes the aerosol source by ultrasonic vibration. The air taking-in flow path 116 is connected to the atomizing section 104, and the air taking-in flow path 116 leads to the outside of the flavor inhaler 100. The aerosol generated in the atomizing section 104 is mixed with air, which is taken via the air taking-in flow path 116, and sent to the aerosol flow path 110.

The flavor source 106 is a unit for providing aerosol with flavor. The flavor source 106 is arranged in a middle position on the aerosol flow path 110. A fluid comprising a mixture of air and the aerosol generated in the atomizing section 104 (in the following description, it should be reminded that this fluid mixture may simply be referred to as aerosol) flows through the aerosol flow path 110 to a mouthpiece (the mouthpiece member 108). That is, in the point of view of the flow of the aerosol, the flavor source 106 is arranged in a position downstream the atomizing section 104. In other words, in the aerosol flow path 110, the position of the flavor source 106 is closer to the mouthpiece than the position of the atomizing section 104. In this manner, the aerosol generated in the atomizing section 104 passes through the atomizing section 104 and arrives at the mouthpiece. When the aerosol passes through the flavor source 106, a flavor component from the flavor source 106 is added to the aerosol. For example, the flavor source 106 may be that which originates from tobacco, such as shredded tobacco, a product which is made by processing raw material comprising tobacco to have a granular form, a sheet form, or a powder form, or the like, or that which does not originate from tobacco, such as a product made by use of a plant other than tobacco (for example, mint, a herb, and so on). For example, the flavor source 106 comprises a nicotine component. The flavor source 106 may comprise a flavor component such as menthol. Note that, in addition to having the flavor source 106, it is possible to make the reservoir 102 to have a material comprising a flavor component. For example, the flavor inhaler 100 may be constructed in such a manner that the flavor source 106 holds flavor material which originates from tobacco and the reservoir 102 comprises flavor material which does not originate from tobacco.

The mouthpiece member 108 is positioned at an end of the aerosol flow path 110 (i.e., positioned downstream the flavor source 106), and constructed to make the aerosol flow path 110 to be opened toward the outside of the flavor inhaler 100. A user takes air including the aerosol into the mouth by holding the mouthpiece member 108 in the user's mouth and inhaling it.

The aerosol flow path 110 is a tubular structure for sending the fluid mixture comprising air and the aerosol generated in the atomizing section 104 to the mouthpiece. As shown in Fig. 1, the aerosol flow path 110 comprises a shared flow path 110C, a first flow path 110A, and a second flow path 110B. The shared flow path 110C connects between the atomizing section 104 and the flow rate adjustment mechanism 112. The aerosol generated in the atomizing section 104 is sent, together with air, to the shared flow path 110C, and to the flow rate adjustment mechanism 112 through the shared flow path 110C. The flow rate adjustment mechanism 112 and the mouthpiece member 108 are connected by two paths, specifically, the first flow path 110A and the second flow path 110B. The flavor source 106 is arranged in a position in the middle of the first flow path 110A. That is, the first flow path 110A connects between the flow rate adjustment mechanism 112 and the flavor source 106, and connects between the flavor source 106 and the mouthpiece member 108. On the other hand, the second flow path 110B connects between the flow rate adjustment mechanism 112 and the mouthpiece member 108 in a direct manner, i.e., without passing through the flavor source 106. In this manner, in the part downstream the flow rate adjustment mechanism 112, the fluid mixture comprising air and the aerosol is divided to flow through the first flow path 110A and the second flow path 110B to be sent to the mouthpiece member 108. A part of the aerosol flowing through the first flow path 110A passes through the flavor source 106 and a flavor component is added thereto, and, thereafter, the part of the aerosol is led to the mouthpiece. Another part of the aerosol flowing through the second flow path 110B does not pass through the flavor source 106; thus, the other part of the aerosol is led to the mouthpiece without addition of the flavor component included in the flavor source 106. The aerosol flown through the first flow path 110A and the aerosol flown through the second flow path 110B are flown into each other at the mouthpiece member 108 and inhaled by a user.

In the flavor inhaler 100 shown as an example in Fig. 1, the flow rate adjustment mechanism 112 is arranged in a position at a branching point in the aerosol flow path 110 where the shared flow path 110C is divided into the first flow path 110A and the second flow path 110B. That is, the starting point (the end of the upstream side) of the second flow path 110B is indirectly connected to the first flow path 110A via the flow rate adjustment mechanism 112. However, the arrangement of the flow rate adjustment mechanism 112 is not limited to that explained above. For example, the flow rate adjustment mechanism 112 may be positioned at a position in the middle of the first flow path 110A (i.e., a position downstream the branching point), or a position in the middle of the second flow path 110B (i.e., a position downstream the branching point). In other words, the starting point of the second flow path 110B may be directly connected to the first flow path 110A at the branching point. In addition, although the first flow path 110A and the second flow path 110B are joined at the mouthpiece member 108 in the flavor inhaler 100 shown as an example in Fig. 1, such a construction is optional. For example, there may be a construction wherein an end (the downstream-side end) of the second flow path 110B is connected to the flavor source 106 (for example, at a position around the center, in terms of direction of flow of the aerosol, of the flavor source 106) instead of the mouthpiece member 108, so that the aerosol flowing through the second flow path 110B passes through a part of the flavor source 106 (for example, a downstream-side half of the flavor source 106). Further, although the first flow path 110A only is provided with the flavor source 106 in the flavor inhaler 100 shown as an example in Fig. 1, a flavor source different from the flavor source 106 (for example, a flavor source which can add a flavor component, which is different from the component which can be added by the flavor source 106, to the aerosol) may further be added to the second flow path 110B.

The flow rate adjustment mechanism 112 is constructed in such a manner that it can adjust the ratio between the flow rate of the fluid flowing from the shared flow path 110C to the first flow path 110A and that of the fluid flowing to the second flow path 110B. As explained above, the fluid flowing through the aerosol flow path 110 is a fluid mixture comprising the aerosol generated in the atomizing section 104 and the air taken from the air taking-in flow path 116. It is supposed that the flow rate of air and the flow rate of aerosol flowing through the shared flow path 110C are $Q_T$ and $M_T$, the flow rate of the air and the flow rate of the aerosol flowing through the first flow path 110A are $Q_1$ and $M_1$, and the flow rate of the air and the flow rate of the aerosol flowing through the second flow path 110B are $Q_2$ and $M_2$, respectively. In this regard, there are conditions that $Q_T=Q_1+Q_2$ and $M_T=M_1+M_2$. The flow rate adjustment mechanism 112 is constructed in such a manner that it can adjust the ratio $\beta$ between the flow rate of the air flowing into the first flow path 110A and that of the air flowing into the second flow path 110B. The air flow rate ratio $\beta$ may be defined as the amount of air flowing through the first flow path 110A to the amount of the whole air flowing through the aerosol flow path 110 (i.e., $\beta=Q_1/Q_T$), or the amount of air flowing through the first flow path 110A to the amount of air flowing through the second flow path 110B (i.e., $\beta=Q_1/Q_2$). In a similar manner, the flow rate ratio $\alpha$ of the aerosol is defined as $\alpha=M_1/M_T$ or $\alpha=M_1/M_2$. Usually, the air flow rate ratio $\beta$ is equal to the aerosol flow rate ratio $\alpha$. The air flow rate ratio $\beta$ (and the aerosol flow rate ratio $\alpha$) is dependent on air-flow resistance of each of the first flow path 110A and the second flow path 110B, and the air-flow resistance is dependent on the length and the cross-sectional area, the degree of bending, the shapes of a branching part and a junction part, and so on of the flow path. The flow rate adjustment mechanism 112 comprises, for example, a structure by which a cross-sectional area of a flow path of at least one of the first flow path 110A and the second flow path 110B can be changed. Note that examples of tangible structures of the flow rate adjustment mechanism 112 will be explained later with reference to Figs. 5-9.

The flavor inhaler 100 can improve the degree of freedom of control of the amount of aerosol and the amount of flavor to be inhaled by a user, by having the flow rate adjustment mechanism 112 explained above. For example, by changing a cross-sectional area of a flow path of one or both of the first flow path 110A and the second flow path 110B, the air flow rate ratio $\beta$ (and the aerosol flow rate ratio $\alpha$) is changed, and, in response thereto, change in the amount of aerosol flowing through the first flow path 110A and the amount of aerosol flowing through the second flow path 110B occur. Further, change in the amount of aerosol flowing through the first flow path 110A leads to change in the amount of the flavor component supplied to a user. Thus, the user can freely adjust the ratio between the aerosol and the flavor component which are to be inhaled.

The flavor inhaler 100 comprises a user setting section 150 which allows a user to set at least one of the air flow rate ratio $\beta$ of the first flow path 110A and the second flow path 110B controlled by the flow rate adjustment mechanism 112 and the amount of aerosol to be generated in the atomizing section 104. For example, the user setting section 150 is constructed as a button, a switch, a control, or the like which can be physically manipulated by a user. As another example, the user setting section 150 may be constructed as a communication interface (for example, a USB terminal or a wireless interface) which receives an instruction from a user via communication connection with an external computer. In the case that the air flow rate ratio $\beta$ of the flow rate adjustment mechanism 112 is set by user manipulation via the user setting section 150, the flow rate adjustment mechanism 112 performs operation in accordance with the setting. In the case that the amount of aerosol to be generated in the atomizing section 104 is set by user manipulation via the user setting section 150, the atomizing section 104 performs operation in accordance with the setting. Setting of the user setting section 150 may be that for one of the flow rate adjustment mechanism 112 and the atomizing section 104, or that for changing operation of both the flow rate adjustment mechanism 112 and the atomizing section 104 at the same time.

In the case that a user wishes to inhale a more (or less) amount of flavor without changing the amount of aerosol to be inhaled, the user performs setting of the user setting section 150 to change the air flow rate ratio of the flow rate adjustment mechanism 112 to make the more (or less) amount of aerosol to be passed through the cartridge 106, and the user does not change the setting of the atomizing section 104. As a result thereof, while a certain amount of aerosol is generated in the atomizing section 104, the air flow rate ratio of the first flow path 110A and the second flow path 110B controlled by the flow rate adjustment mechanism 112 is changed according to the user manipulation. Thus, the flavor inhaler 100 can change the amount of flavor supplied to the user, while maintaining the amount of aerosol supplied to the user to be constant. Another example is that, in the case that the user setting section 150 is set for changing one of the air flow rate ratio of the flow rate adjustment mechanism 112 and the amount of aerosol to be generated in the atomizing section 104, the operation of another of the flow rate adjustment mechanism 112 and the atomizing section 104 may be changed according to controlling (a first mode of control and a second mode od control) by a control section 130 which will be explained later.

The flavor inhaler 100 according to the present embodiment further comprises the control section 130 and a memory 140. The control section is an electronic circuit module constructed as a microprocessor or a microcomputer, and is programmed to control operation of the flavor inhaler 100 according to computer-executable instructions stored in the memory 140. The memory comprises an information storing medium such as a ROM, a RAM, a flash memory, or the like. The memory 140 stores, in addition to the computer-executable instructions, setting data which are necessary for controlling the flavor inhaler 100.

In rough outline, the control section 130 is constructed to control operation of at least one of the flow rate adjustment mechanism 112 and the atomizing section 104. In a first mode of operation, the control section 130 controls the air flow rate ratio $\beta$ between the first flow path 110A and the second flow path 110B, by controlling the flow rate adjustment mechanism 112 based on the amount $M_T$ of aerosol generated in the atomizing section 104. In a second mode of operation, the control section 130 controls the amount $M_T$ of aerosol generated in the atomizing section 104, based on the air flow rate ratio $\beta$ between the first flow path 110A and the second flow path 110B controlled by the flow rate adjustment mechanism 112. In each of the fist and second modes of operation, the state of operation of one of the atomizing section 104 and the flow rate adjustment mechanism 112 affects operation of another of them. Further, in a third mode of operation, the control section 130 performs control based on an accumulated value of the amounts of aerosol generated in the atomizing section 104. Details of control of each mode will be explained in the following description.

< First mode of control >

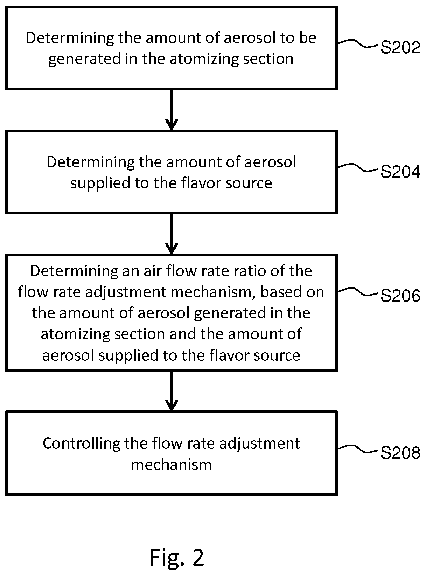

Fig. 2 is a flow chart showing operation of a control section 130 relating to the first mode of control. In step S202, the control section 130 determines the amount $M_T$ of aerosol to be generated in the atomizing section 104. Determining the amount $M_T$ of aerosol to be generated includes reading, from the memory 140, a set value of the amount of aerosol to be generated in the atomizing section 104 (a first example), and estimating the amount of aerosol, which will be actually generated, based on electric energy supplied to the heater of the atomizing section 104 (a second example). The amount $M_T$ of aerosol generated in the atomizing section 104 is equal to a set value of the amount of aerosol that should be generated in the atomizing section 104 (an instructed value for the atomizing section 104) in the first example, and is equal to an estimate value of the amount of aerosol that is expected to be generated in the atomizing section 104 in the second example.

In the first example, an initial set value of the amount of aerosol, that should be generated by the atomizing section 104 when starting operation of the flavor inhaler 100 (when starting inhaling action), is stored in the memory 140 in advance. Also, in the case that the amount of aerosol that should be generated by the atomizing section 104 is newly set via the user setting section 150, the initial set value of the amount of aerosol to be generated, that is stored in the memory 140, is updated by the new set value. The control section 130 obtains an initial set value of the amount of aerosol to be generated or a new set value from the memory 140, and decides to use the initial set value or the new set value as the amount $M_T$ of aerosol to be generated in the atomizing section 104.

In the second example, the control section 130 estimates the amount of aerosol, which will be generated in the atomizing section 104, based on the electric energy supplied to the heater of the atomizing section 104. Usually, the amount of aerosol generated in the atomizing section 104 is determined according to energy supplied to the aerosol source. For example, relationship between the electric energy supplied to the heater of the atomizing section 104 and the estimated amounts of aerosol generated from the aerosol source when the heater is heated by the electric energy is stored in the memory 140 in advance. The control section 130 observes the electric energy supplied from the battery 114 to the heater of the atomizing section 104, obtains an estimate value of the amount of aerosol to be generated, that corresponds to an observed value, from the memory 140, and decides to use the obtained estimate value as the amount $M_T$ of aerosol generated in the atomizing section 104.

Note that, regarding the electric energy for the heater, the electric energy may be calculated by measuring a resistance value of the heater, a voltage applied to the heater, and the time of electric conduction to the heater, and using the measured values, or the electric energy may be estimated by measuring a voltage applied to the heater and the time of electric conduction to the heater, under an assumption that the resistance value of the heater does not change, and using the measured values. Under the condition that electric power supplied to the heater per unit time is constant, the control section 130 may observe the time of electric conduction to the heater (for example, electric conduction time per single inhaling action), instead of observing the electric energy (= electric power * electric conduction time) supplied to the heater. In the case that it is necessary to consider voltage reduction of the battery 114, the control section 130 observes both instantaneous electric power supplied to the heater and electric conduction time. In this regard, for compensating for voltage reduction of the battery 114, a duty ratio may be adjusted to extend an electric conduction cycle (an ON period), in pulse width modulation (PWM) control for the heater.

In step S204, the control section 130 determines the amount $M_1$ of aerosol to be passed through the flavor source 106. For example, reference values of amounts of aerosol which are required for taking out desired amounts of flavor to be included in the aerosol are stored in the memory 140 in advance. The control section 130 obtains a reference value, and decides to use it as the amount $M_1$ of aerosol which should be passed through the flavor source 106. The flavor inhaler 100 may be constructed in such a manner that there are plural types of cartridges which hold different flavor sources 160 and are replaceable. In such a case, the flavor inhaler 100 further comprises a mechanism for identifying the type of cartridge (i.e., the flavor source 106) presently installed, and the memory 140 may store different reference values of the amounts of aerosol for respective various flavor sources 160. The control section 130 obtains a reference value corresponding to the identified flavor source 106 from the memory 140, and decides to use the obtained reference value as the amount $M_1$ of aerosol which should be passed through the flavor source 106.

Note that step S204 may be performed before performing step 202, or step S204 and step S202 may be performed at the same time (in parallel).

In step S206, the control section 130 determines the air flow rate ratio $\beta$ of the flow rate adjustment mechanism 112, based on the amount $M_T$ of the aerosol generated in the atomizing section 104 and the amount $M_1$ of the aerosol to be passed through the flavor source 106. When the amount $M_T$ of the aerosol generated in the atomizing section 104 is changed, the control section 130 changes, in response to the change, the air flow rate ratio $\beta$ of the flow rate adjustment mechanism 112. For example, in the case that the amount $M_T$ of the aerosol generated in the atomizing section 104 is made to be large, the control section 130 changes the air flow rate ratio $\beta$ of the flow rate adjustment mechanism 112 to have a small value, and, in the case that the amount $M_T$ of the aerosol generated in the atomizing section 104 is made to be large, the control section 130 changes the air flow rate ratio $\beta$ of the flow rate adjustment mechanism 112 to have a large value. For example, the control section 130 uses the amount $M_T$ of the aerosol generated in the atomizing section 104 and the amount $M_1$ of the aerosol to be passed through the flavor source 106 to determine the air flow rate ratio $\beta$ of the flow rate adjustment mechanism 112 by use of following formula (1).

$$(1) \quad \beta = \alpha = M_1/M_T$$

In step S208, the control section 130 controls the flow rate adjustment mechanism 112 in such a manner that the ratio $Q_1/Q_T$ of the flow rate $Q_1$ of air flowing through the first flow path 110A to the total flow rate $Q_T$ of air flowing through the aerosol flow path 110 coincides with the air flow rate ratio $\beta$ determined in step S206. For example, the control section 130 controls the air flow rate ratio of the flow rate adjustment mechanism 112 to have a desired value, by operating the flow rate adjustment mechanism 112 to change a cross-section area of the flow path of at least one of the first flow path 110A and the second flow path 110B. The control section 130 may detect, by use of a flow rate sensor 124, at least one of the flow rate $Q_1$ of air flowing through the first flow path 110A and the flow rate $Q_2$ of air flowing through the second flow path 110B, and performs feedback control of the air flow rate ratio of the flow rate adjustment mechanism 112 by use of the detected flow rate(s). The flow rate adjustment mechanism 112 is electrically connected to the battery 114, and operates to change a cross-section area of the flow path of at least one of the first flow path 110A and the second flow path 110B in accordance with instructions from the control section 130, for example.

As explained above, as a result that the control section 130 performs the first mode of control, the flavor inhaler 100 operates in such a manner that the air flow rate ratio of the flow rate adjustment mechanism 112 is adjusted to correspond to the amount of aerosol generated in the atomizing section 104.

For example, at the time of start of the operation of the flavor inhaler 100, the atomizing section 104 generates a certain amount of aerosol in accordance with an initial set value of the amount of aerosol to be generated, wherein the initial set value has been stored in the memory 140 in advance. The control section 130 obtains an initial set value of the amount of aerosol to be generated and a reference value of the amount of aerosol to be passed through the flavor source 106 from the memory 140, calculates the air flow rate ratio $\beta$ of the flow rate adjustment mechanism 112, based on these values and the above formula (1), and controls the flow rate adjustment mechanism 112 in accordance with the calculated air flow rate ratio $\beta$. As a result thereof, the aerosol of the amount $M_1$ flows through the first flow path 110A and passes through the flavor source 106.

Further, in the case that setting of the amount of aerosol to be generated is changed via the user setting section 150, the atomizing section 104 generates the amount of aerosol indicated by the changed set value. The control section 130 obtains, from the memory 140, the updated set value of the amount of aerosol to be generated and the reference value of the amount of aerosol to be passed through the flavor source 106, calculates the air flow rate ratio $\beta$ of the flow rate adjustment mechanism 112 based on the above obtained values and above formula (1), and controls the flow rate adjustment mechanism 112 in accordance with the calculated air flow rate ratio $\beta$. As a result thereof, the amount of aerosol that is different from the amount at the time of start of operation of the flavor inhaler 100 is generated in the atomizing section 104, and the amount $M_1$ of the aerosol that is equal to the amount at the time of start of operation of the flavor inhaler 100 is sent to flow though the first flow path 110A and passed through the flavor source 106.

Also, in the case that the electric energy supplied from the battery 114 to the heater of the atomizing section 104 changes (for example, due to voltage reduction of the battery 114, or the like), the amount of aerosol generated in the atomizing section 104 also changes. The control section 130 detects the electric energy supplied to the atomizing section 104, obtains, from the memory 140, the value of the amount of aerosol to be generated, which corresponds to the above detected value, and the reference value of the amount of aerosol sent to be passed through the flavor source 106, calculates the air flow rate ratio $\beta$ of the flow rate adjustment mechanism 112 based on the above obtained values and above formula (1), and controls the flow rate adjustment mechanism 112 in accordance with the calculated air flow rate ratio $\beta$. As a result thereof, the amount $M_1$ of the aerosol that is equal to the amount at the time of start of operation of the flavor inhaler 100 is sent to flow though the first flow path 110A and passed through the flavor source 106, independent of change in the amount of electric power supplied to the atomizing section 104.

In this manner, in the example of the first mode of control, the control section 130 controls the air flow rate ratio of the flow rate adjustment mechanism 112 in such a manner that the amount of the aerosol flowing though the first flow path 110A is made to be constant. Thus, the flavor inhaler 100 can supply a constant amount of flavor to a user, independent of the amount of aerosol generated in the atomizing section. In this regard, the first mode of control is not limited to controlling for precisely maintaining the values of the amounts of the aerosol and the flavor to be the same values. For example, in the case that the amount of aerosol generated in the atomizing section 104 is increased or decreased, change in the amount of the aerosol flowing though the first flow path 110A can be reduced, compared with the degree of change in the amount of the generated aerosol, by performing control to make the air flow rate ratio $\beta$ of the flow rate adjustment mechanism 112 (the ratio of distribution to the first flow path 110A) smaller or larger to some extent.

< Second mode of control >

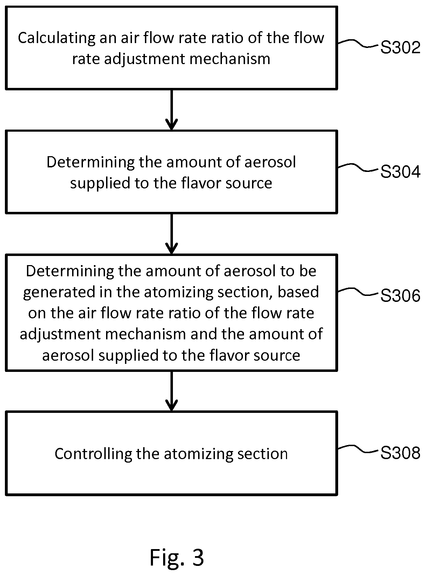

Fig. 3 is a flow chart showing operation of the control section 130 relating to a second mode of control. In step S302, the control section 130 calculates the air flow rate ratio $\beta$ of the flow rate adjustment mechanism 112, based on a state of operation of the flow rate adjustment mechanism 112 (for example, the cross-section areas of flow paths of the first flow path 110A and the second flow path 110B). For example, the flow rate adjustment mechanism 112 is constructed in such a manner that the first flow path 110A has a flow-path cross-section area that is fixed, and the second flow path 110B has a flow-path cross-section area that can be varied in accordance with user manipulation via the user setting section 150. The flow-path cross-section area of the second flow path 110B is represented by a ratio (a degree of opening) X to the flow-path cross-section area when the second flow path 110B is fully opened. The degree of opening X of the second flow path 110B is a parameter representing the state of operation of the flow rate adjustment mechanism 112. Relationship between the degrees of opening X of the second flow path 110B and the air flow rate ratios $\beta$ of the flow rate adjustment mechanism 112 is stored in the memory 140 in advance. The control section 130 detects the degree of opening X of the second flow path 110B, and obtains, from the memory 140, the value of the air flow rate ratio $\beta$ of the flow rate adjustment mechanism 112 corresponding to the above detected value. For example, a variable resistor, which operates in response to change in the flow-path cross-section area, is attached to the second flow path 110B, and the control section 130 can detect the degree of opening X of the second flow path 110B based on a resistance value of the variable resistor. In another example, an optical sensor for detecting the degree of opening X may be used. The flow rate adjustment mechanism 112 may be constructed in such a manner that both the first flow path 110A and the second flow path 110B have flow-path cross-section areas that can be varied in accordance with user manipulation via the user setting section 150. In such a case, relationship between the degrees of opening $X_1$ of the first flow path 110A and the degrees of opening $X_2$ of the second flow path 110B and the air flow rate ratios $\beta$ of the flow rate adjustment mechanism 112 is stored in the memory 140 in advance. The control section 130 detects the degree of opening $X_1$ of the first flow path 110A and the degree of opening $X_2$ of the second flow path 110B, and obtains, from the memory 140, the value of the air flow rate ratio $\beta$ of the flow rate adjustment mechanism 112 corresponding to the above detected values.

In step S304, the control section 130 determines the amount $M_1$ of aerosol to be passed through the flavor source 106. This step S304 is the same as step S204 in the above first mode of control. Also, similar to the case of the first mode of operation, step S304 may be performed before performing step 302, or step S304 and step S302 may be performed at the same time (in parallel).

In step S306, the control section 130 determines the amount $M_T$ of aerosol to be generated in the atomizing section 104, based on the air flow rate ratio $\beta$ of the flow rate adjustment mechanism 112 and the amount $M_1$ of aerosol to be passed through the flavor source 106. When the air flow rate ratio $\beta$ of the flow rate adjustment mechanism 112 is changed in accordance with user manipulation via the user setting section 150, the control section 130 responds thereto to change the amount $M_T$ of aerosol generated in the atomizing section 104. For example, in the case that the air flow rate ratio $\beta$ of the flow rate adjustment mechanism 112 is changed to make it large by user manipulation, the control section 130 makes the amount $M_T$ of aerosol generated in the atomizing section 104 small, and, in the case that the air flow rate ratio $\beta$ of the flow rate adjustment mechanism 112 is changed to make it small, the control section 130 makes the amount $M_T$ of aerosol generated in the atomizing section 104 large. For example, the control section 130 uses the air flow rate ratio $\beta$ of the flow rate adjustment mechanism 112 and the amount $M_1$ of the aerosol to be passed through the flavor source 106 to determine the amount $M_T$ of aerosol to be generated in the atomizing section 104 by use of following formula (2).

$$(2) \quad M_T = M_1/\beta$$

In step S308, the control section 130 controls the atomizing section 104 in such a manner that the amount of aerosol to be generated in the atomizing section 104 becomes equal to the amount $M_T$ of aerosol determined in step S306. The control section 130 controls the amount of aerosol to be generated in the atomizing section 104 to have a desired value, by changing the electric energy supplied from the battery 114 to the heater of the atomizing section 104. For example, relationship between the electric energy supplied to the heater of the atomizing section 104 and the amounts of aerosol generated when the heater is heated by the electric energy is stored in the memory 140 in advance. The control section 130 obtains the value of electric energy, which is to be suppled to the heater, corresponding to the amount $M_T$ of aerosol determined in step S306 from the memory 140, and performs control in such a manner that the electric energy suppled to the heater of the atomizing section 104 coincides with the above obtained value.

As explained above, as a result that the control section 130 performs the second mode of control, the flavor inhaler 100 operates in such a manner that the amount of aerosol generated in the atomizing section 104 is adjusted in accordance with the air flow rate ratio of the flow rate adjustment mechanism 112.

For example, when starting operation of the flavor inhaler 100, the flow rate adjustment mechanism 112 has already set in such a manner that the degree of opening X of the second flow path 110B takes a predetermine value. The control section 130 detects the degree of opening X of the second flow path 110B, obtains, from the memory 140, the value of the air flow rate ratio of the flow rate adjustment mechanism 112, which corresponds to the above detected value, and the reference value of the amount of aerosol to be passed through the flavor source 106, calculates the amount $M_T$ of aerosol to be generated in the atomizing section 104 based on the above values and above formula (2), and controls the atomizing section 104 in accordance with the above calculated amount $M_T$ of aerosol to be generated. As a result thereof, the aerosol of the amount $M_T$ flows through the first flow path 110A and passes through the flavor source 106.

Further, in the case that the air flow rate ratio of the flow rate adjustment mechanism 112 is changed via the user setting section 150, the control section 130 detects the changed degree of opening X of the second flow path 110B, obtains, from the memory 140, the value of the air flow rate ratio of the flow rate adjustment mechanism 112, which corresponds to the new degree of opening, and the reference value of the amount of aerosol to be passed through the flavor source 106, calculates the amount $M_T$ of aerosol to be generated in the atomizing section 104 based on the above values and above formula (2), and controls the atomizing section 104 in accordance with the above calculated amount $M_T$ of aerosol to be generated. As a result thereof, the amount of aerosol that is different from the amount at the time of start of operation of the flavor inhaler 100 is generated in the atomizing section 104, and the amount $M_1$ of the aerosol that is equal to the amount at the time of start of operation of the flavor inhaler 100 is sent to flow though the first flow path 110A and passed through the flavor source 106.

In this manner, in the example of the second mode of control, the control section 130 controls the amount of aerosol to be generated in the atomizing section 104 in response to change in the air flow rate ratio of the flow rate adjustment mechanism 112, that is made by user manipulation, in such a manner that the amount of the aerosol flowing though the first flow path 110A is made to be constant. Thus, the flavor inhaler 100 can supply a constant amount of flavor to a user, independent of the state of operation of the flow rate adjustment mechanism 112. In this regard, the second mode of control is not limited to controlling for precisely maintaining the values of the amounts of the aerosol and the flavor to be the same values. For example, in the case that the air flow rate ratio of the flow rate adjustment mechanism 112 (the ratio of distribution to the first flow path 110A) is made larger or smaller by user manipulation applied to the flow rate adjustment mechanism 112, change in the amount of the aerosol flowing though the first flow path 110A can be reduced, by performing control to make the amount of aerosol to be generated in the atomizing section 104 smaller or larger to some extent.

< Third mode of operation >

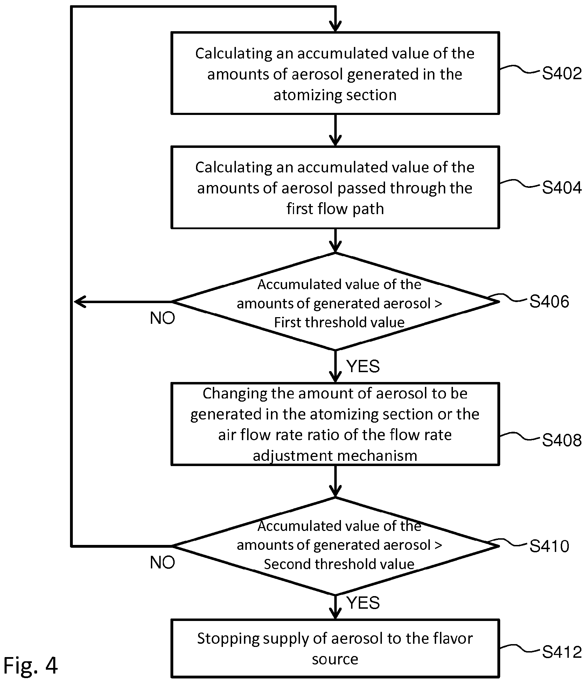

Fig. 4 is a flow chart showing operation of the control section 130 relating to a third mode of control. In step S402, the control section 130 calculates an accumulated value of the amounts of aerosol generated in the atomizing section 104. As explained above, usually, the amount of aerosol to be generated is determined based on energy to be supplied to the aerosol source. For example, relationship between the electric energy supplied to the heater of the atomizing section 104 and the amounts of aerosol generated from the aerosol source when the heater is heated by the electric energy is stored in the memory 140 in advance. The control section 130 observes, over time, the electric energy (= electric power * electric conduction time) supplied from the battery 114 to the heater of the atomizing section 104, obtains the values of amounts of generated aerosol that correspond to the above observed values, respectively, and adds the above obtained values to thereby obtain, in an estimation manner, an accumulated value of the amounts of the aerosol generated in the atomizing section. In the case that it is necessary to consider voltage reduction of the battery 114, the control section 130 observes both instantaneous electric power supplied to the heater and electric conduction time. Under the condition that electric power supplied to the heater per unit time is constant, the control section 130 may observe time of electric conduction to the heater instead of observing electric energy supplied to the heater, and obtain the accumulated value of the amounts of generated aerosol based on the accumulated value of the electric conduction time. Note that the accumulated value of the amounts of aerosol may be that during a single inhaling period, or the accumulated value of the amounts of aerosol of respective inhaling periods over plural inhaling periods.

In step S404, the control section 130 calculates an accumulated value of the amounts of aerosol passed through the first flow path 110A. The amount of aerosol passed through the first flow path 110A can be calculated from the amount of aerosol generated in the atomizing section 104 and the air flow rate ratio (= the aerosol flow rate ratio) of the flow rate adjustment mechanism 112. The control section 130 observes, over time, the air flow rate ratios of the flow rate adjustment mechanism 112 (for example, the degrees of opening X), successively calculates the amounts of aerosol passed through the first flow path 110A, based on the air flow rate ratios at respective points in time and the corresponding amounts of generated aerosol at the respective points in time, that are successively obtained from the memory 140 in above step S402, and adds the above obtained values to thereby obtain an accumulated value of the amounts of aerosol passed through the first flow path 110A. The control section 130 may be constructed to calculate the accumulated value of the amounts of aerosol passed through the second flow path 110B, in a manner similar to that explained above. Note that step S404 is optional, thus, it may be omitted.

In step S406, the control section 130 judges whether the accumulated value of the amounts of aerosol generated in the atomizing section 104 exceeds a first threshold value. If the accumulated value of the amounts of generated aerosol exceeds the first threshold value, the process proceeds to step S408, and, if not, the process returns to step S402. In the case that an accumulated value of the amounts of aerosol passed through the first flow path 110A is calculated in step S404, the control section 130 may be constructed in such a manner that it judges whether the accumulated value of the amounts of aerosol passed through the first flow path 110A exceeds a predetermined threshold value that corresponds to the above first threshold value, instead of judging whether the accumulated value of the amounts of aerosol generated in the atomizing section 104 exceeds the above first threshold value. The judgment in step S406 may be performed at any timing such as that 1) after single inhaling action is completed, 2) during a predetermined time lag that is between a point in time when inhaling action is detected by the inhaling sensor 122 and a point in time when atomizing of aerosol is started, or 3) during inhaling action (during a period of electric conduction to the heater), for example.

In step S408, the control section 130 performs control that is applied to the atomizing section 104 or the flow rate adjustment mechanism 112, for changing the amount of aerosol to be generated in the atomizing section 104 or the air flow rate ratio of the flow rate adjustment mechanism 112. For example, the control section 130 controls the atomizing section 104 or the flow rate adjustment mechanism 112 for changing the amount of aerosol passing through the first flow path 110A. There may be a case that ability to release a flavor component of the flavor source 106 is gradually degraded due to flow of aerosol passing through it. For compensating for lowering of the amount of flavor released from the flavor source 106, control for increasing the amount of aerosol passing through the first flow path 110A is applied. In such a case, the first threshold value used in judgment performed in step S406 corresponds to an accumulated amount of aerosol that is sufficient for consuming a certain amount of the flavor component from the flavor source 106. For example, the control section 130 controls the atomizing section 104 to increase the amount of aerosol to be generated in the atomizing section 104, without changing the air flow rate ratio of the flow rate adjustment mechanism 112, to thereby increase the amount of aerosol passing through the first flow path 110A. As another example, it may be possible to construct the control section 130 in such a manner that it controls the flow rate adjustment mechanism 112 to make the air flow rate ratio of the flow rate adjustment mechanism 112 (the ratio of distribution to the first flow path 110A) large, without changing the amount of aerosol to be generated in the atomizing section 104, to thereby increase the amount of aerosol passing through the first flow path 110A. As a result thereof, the flavor inhaler 100 can suppress effect due to consumption of the flavor source, and supply a certain amount of flavor to a user.

In step S410, the control section 130 judges whether the accumulated value of the amounts of aerosol generated in the atomizing section 104 exceeds a second threshold value. If the accumulated value of the amounts of generated aerosol exceeds the second threshold value, the process proceeds to step S412, and, if not, the process returns to first step S402. The second threshold value is set to a value larger than the above first threshold value. In the case that an accumulated value of the amounts of aerosol passed through the first flow path 110A is calculated in step S404, the control section 130 may be constructed in such a manner that it judges whether the accumulated value of the amounts of aerosol passed through the first flow path 110A exceeds a predetermined threshold value that corresponds to the above second threshold value, instead of judging whether the accumulated value of the amounts of aerosol generated in the atomizing section 104 exceeds the above second threshold value.

In step S412, the control section 130 performs control to stop supply of aerosol to the flavor source 106. For example, the control section 130 controls the flow rate adjustment mechanism 112 in such a manner that it blocks communication between the atomizing section 104 and the first flow path 110A (for example, by setting the degree of opening $X_1$ of the first flow path 110A to zero). Also, the control section 130 may control the flow rate adjustment mechanism 112 in such a manner that it blocks communication between the atomizing section 104 and both the first flow path 110A and the second flow oath 110B. In a further example, the control section 130 may control the atomizing section 104 in such a manner that supply of electric power from the battery 114 to the heater of the atomizing section 104 is cut off. As a result thereof, the flavor inhaler 100 can prevent excessive supply of flavor to a user and supply of aerosol that does not contain sufficient amount of flavor.

In a manner similar to that in above step S406, the judgment in step S410 may be performed at any timing such as that 1) after single inhaling action is completed, 2) during a predetermined time lag that is between a point in time when inhaling action is detected by the inhaling sensor 122 and a point in time when atomizing of aerosol is started, or 3) during inhaling action (during a period of electric conduction to the heater), for example. Regarding the timing in the above example, in the case that judgment in step S410 is performed at timing 1) after single inhaling action is completed, an unnatural feel sensed by a user can be suppressed, since the control performed in step S412 does not interrupt atomizing of aerosol during inhaling action of the user or does not block the flow path.

Note that the order of the judgment process in step S406 and the control process in step S408 following step S406, and the judgment process in step S410 and the control process in step S412 following step S410 may be changed between them and performed in the changed order.

Next, more tangible constructions of the flavor inhalers will be explained.

Fig. 5 is a figure showing a construction of a flavor inhaler 500 according to an embodiment. The flavor inhaler 500 has a shape which extends in a predetermined direction A that is a direction from a non-mouthpiece end to a mouthpiece end. As shown in Fig. 5, the flavor inhaler 500 comprises an inhaler body 510, a mouthpiece member 520, and a cartridge 530. The mouthpiece member 520 and the cartridge 530 correspond to the mouthpiece member 108 and the flavor source 106 in Fig. 1, respectively.

A main body of the flavor inhaler 500 comprises the inhaler body 510 which has a shape allowing the cartridge 530 to be connected thereto. The inhaler body 510 comprises an atomizing unit 511 which atomizes an aerosol source without a burning process. The inhaler body 510 further comprises a battery assembly, a control section, and a memory which are not shown in the figure. The battery assembly comprises a battery. The control section may be positioned in the inside of the battery assembly, for example. The battery, the control section, and the memory correspond to the battery 114, the control section 130, and the memory 140 in Fig. 1, respectively.

The atomizing unit 511 comprises a reservoir 511P, a wick 511Q, and an atomizing section 511R. The reservoir 511P holds an aerosol source. For example, the reservoir 511P is a porous medium comprising non-tobacco material such as a resin web and so on. The wick 511Q sucks the aerosol source held in the reservoir 511P. For example, the wick 511Q comprises glass fibers. The atomizing section 511R atomizes the aerosol source sucked by the wick 511Q. For example, the atomizing section 511R comprises a heating wire wound, with a predetermined pitch, around the wick 511Q. The reservoir 511P and the atomizing section 511R correspond to the reservoir 102 and the atomizing section 104 in Fig. 1, respectively.

The mouthpiece member 520 comprises a mouthpiece which is to be held in the user's mouth and constructed to be attachable/detachable to/from the inhaler body 510. For example, the mouthpiece member 520 is attached to the inhaler body 510 in a screwing or mating manner.

The cartridge 530 is an example of a flavor source unit which is constructed to be connectable to the inhaler body 510 which is a component of the flavor inhaler 500. The cartridge 530 is arranged in a position closer to the mouthpiece side than the position of the atomizing unit 511, in the flow path of a gas (hereinafter, air) inhaled from the mouthpiece. In other words, it is not necessarily arrange, in terms of a physical space, the cartridge 530 in a position closer to the mouthpiece side than the position of the atomizing unit 511, and it is satisfactory if the cartridge 530 is arranged in a position closer to the mouthpiece side than the position of the atomizing unit 511 in the aerosol flow path which leads the aerosol generated in the atomizing unit 511 to the mouthpiece side.

Specifically, the cartridge 530 comprises a cartridge body 531, a flavor source 532, and a mesh 533 (a mesh 533A and a mesh 533B).

The cartridge body 531 has a cylindrical shape which extends in the predetermined direction A. The cartridge body 531 houses the flavor source 532.

The flavor source 532 is arranged in a position closer to the mouthpiece side than the position of the atomizing unit 511 in the flow path of the air inhaled from the mouthpiece. The flavor source 532 provides the aerosol generated in the aerosol source with fragrance inhaling taste. In other words, the flavor added to the aerosol by the flavor source 532 is supplied to the mouthpiece.

The flavor source 532 comprises an ingredient piece which provides aerosol generated in the atomizing unit 511 with fragrance inhaling taste. Shredded tobacco or a product, which is made by processing raw material comprising tobacco to have a granular form, may be used as an ingredient piece which is a component of the flavor source 532. In this regard, the flavor source 532 may comprise a product which is made by processing raw material comprising tobacco to have a sheet form. Also, the ingredient piece, which is a component of the flavor source 532, may comprise a plant other than tobacco (for example, mint, a herb, and so on). The flavor source 532 may be provided with flavor such as menthol or the like.

With respect to the flavor source 532, the mesh 533A is positioned to cover an opening of the cartridge body 531 at the non-mouthpiece side, and, with respect to the flavor source 532, the mesh 533B is positioned to cover an opening of the cartridge body 531 at the mouthpiece side. Each of the mesh 533A and the mesh 533B has a degree of coarseness that is sufficient to prevent the ingredient piece, which is a component of the flavor source 532, from passing through the mesh.

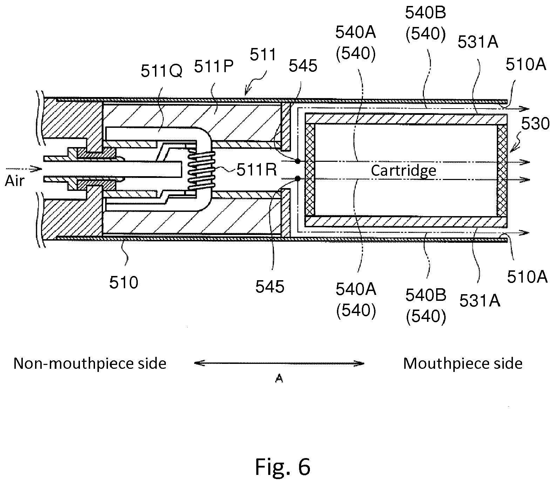

Fig. 6 is a figure showing an aerosol flow path of the flavor inhaler 500. Specifically, Fig. 6 is a schematic cross-section view showing an internal structure of the flavor inhaler 500 in a state that the cartridge 530 is housed in the inhaler body 510.

As shown in Fig. 6, the flavor inhaler 500 comprises an aerosol flow path 540 which guides the aerosol generated in the atomizing unit 511 to the mouthpiece side. In other words, in the state that the cartridge 530 is housed in the inhaler body 510, the aerosol flow path 540, which guides the aerosol generated in the atomizing unit 511 to the mouthpiece side, is formed. The aerosol flow path 540 comprises a first flow path 540A which guides the aerosol to the mouthpiece side via the flavor source 532, and a second flow path 540B which is different from the first flow path 540A. The second flow path 540B is that which guides the aerosol to the mouthpiece side without passing through the flavor source 532. The aerosol flow path 540, the first flow path 540A, and the second flow path 540B correspond to the aerosol flow path 110, the first flow path 110A, and the second flow path 110B in Fig. 1, respectively.

In a cross section perpendicular to the predetermined direction A, the outer diameter of the cartridge body 531 is smaller than the inner diameter of the inhaler body 510. Also, the second flow path 540B is formed between an outer surface 531A of the cartridge body 531 and an inner surface 510A of the inhaler body 510. Further, a branching section 545 for the first flow path 540A

and the second flow path 540B is arranged at a position outside the cartridge body 531.

In this manner, the first flow path 540A is formed inside the cartridge body 531, and the second flow path 540B is formed outside the cartridge body 531.

Note that the first flow path 540A and the second flow path 540B have a shared flow path which is common to them. The above explained branching section 545 is formed in the shared flow path which is formed between the atomizing unit 511 and the cartridge 530. The shared flow path corresponds to the shared flow path 110C in Fig. 1.

The branching section 545 for the first flow path 540A and the second flow path 540B is provided with an orifice which is not shown in the figure. The orifice corresponds to the flow rate adjustment mechanism 112 in Fig. 1. The orifice is constructed in such a manner that it is controlled by the control section to change a cross-section area of a flow path of at least one of the first flow path 540A and the second flow path 540B.

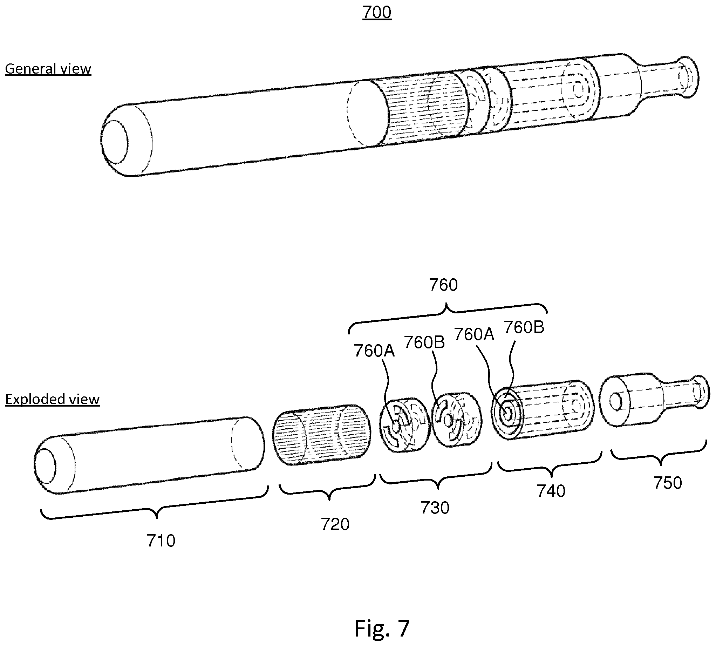

Fig. 7 is a figure showing a construction of a flavor inhaler 700 according to another embodiment. The flavor inhaler 700 comprises a battery assembly 710, an atomizing section 720, a flow rate adjustment mechanism 730, a cartridge 740, and a mouthpiece member 750. The battery assembly 710 further comprises, within it, a battery, a reservoir, a control section, and a memory which are not shown in the figure. Generally, the flavor inhaler 700 has an elongated cylindrical shape as a whole. The battery assembly 710 is constructed to be attachable/detachable to/from the atomizing section 720. The atomizing section 720, the flow rate adjustment mechanism 730, the cartridge 740, and the mouthpiece member 750 correspond to the atomizing section 104, the flow rate adjustment mechanism 112, the flavor source 106, and the mouthpiece member 108 in Fig. 1, respectively. Also, the battery, the reservoir, the control section, and the memory in the battery assembly 710 correspond to the battery 114, the reservoir 102, the control section 130, and the memory 140 in Fig. 1, respectively.

The flavor inhaler 700 further comprises an aerosol flow path 760. The atomizing section 720, the flow rate adjustment mechanism 730, the cartridge 740, and the mouthpiece member 750 are arranged in this order from an upstream side to a downstream side. In a state that the flavor inhaler 700 has been assembled (the general view in Fig. 7), the aerosol flow path 760 connects between the atomizing section 720 at the upstream side and the mouthpiece member 750 at the downstream side. The aerosol flow path 760 comprises a first flow path 760A and a second flow path 760B. The first flow path 760A comprises a hollow which has been bored though, in a longitudinal direction, a center part of the flow rate adjustment mechanism 730, and a hollow which has been bored through, in a longitudinal direction, a center part of the cartridge 740. The second flow path 760B comprises a hollow which has been bored through, in a longitudinal direction, a peripheral part of the flow rate adjustment mechanism 730, and a hollow which has been bored through, in a longitudinal direction, a peripheral part of the cartridge 740. In the state that the flavor inhaler 700 has been assembled, the hollow in the center part of the flow rate adjustment mechanism 730 and the hollow in the center part of the cartridge 740 form an integrated continuous hollow which is a first flow path 760A, and the hollow in the peripheral part of the flow rate adjustment mechanism 730 and the hollow in the peripheral part of the cartridge 740 form an integrated continuous hollow which is a second flow path 760B. The cartridge 740 holds a flavor source in a part of the hollow in the center part, i.e., a part which is a component of the first flow path 760A. The hollow in the peripheral part of the cartridge 740, i.e., the part which is a component of the second flow path 760B does not hold a flavor source. In this manner, the aerosol generated in the atomizing section 720 is divided to flow through the first flow path 760A and the second flow path 760B and supplied to the mouthpiece member 750. A part (a first part) of the aerosol flown into the first flow path 760A passes through the flavor source in the cartridge 740 and is led to the mouthpiece. Another part (a second part) of the aerosol flown into the second flow path 760B is led to the mouthpiece without passing through the flavor source. The aerosol from the first flow path 760A and the aerosol from the second flow path 760B flow into each other at the mouthpiece member 750 and the resultant aerosol is absorbed by a user. The aerosol flow path 760, the first flow path 760A, and the second flow path 760B correspond to the aerosol flow path 110, the first flow path 110A, and the second flow path 110B in Fig. 1, respectively.

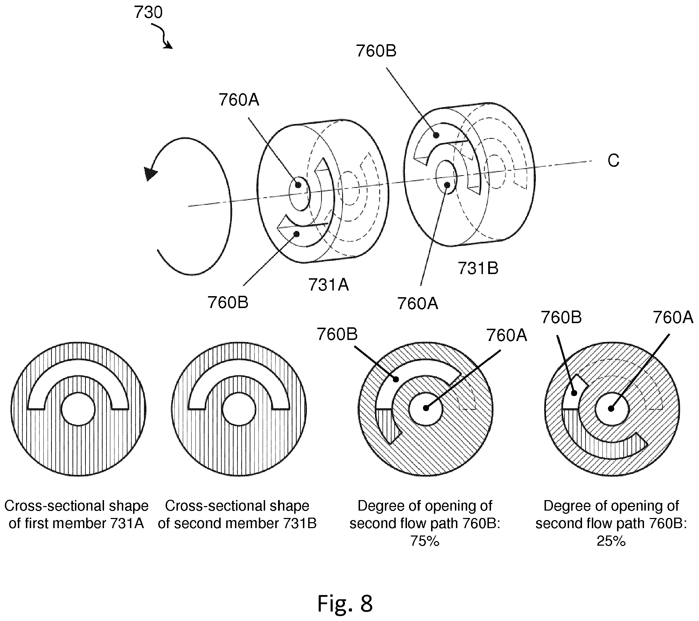

Fig. 8 is a figure showing an example of a construction of a flow rate adjustment mechanism 730 and operation thereof. The flow rate adjustment mechanism 730 comprises a first member 731A and a second member 731B. The first member 731A and the second member 731B have cylindrical shapes, and are arranged to be aligned on a shared center axis C. Both the first member 731A and the second member 731B, or one of them, are/is constructed in such a manner that they/it can be rotated about the center axis C as the axis of rotation. The first member 731A and the second member 731B have hollows which have been bored through, in longitudinal directions, center parts thereof. In the state that the flavor inhaler 700 has been assembled, the hollows in the center parts of the first member 731A and the second member 731B form a part of the first flow path 760A. The shapes of cross sections, which are perpendicular to the center axis C, of the hollows in the center parts are circular, have the same size, and share the center axis C as that defining the centers thereof. Thus, when the first member 731A and the second member 731B are relatively rotated about the center axis C as the axis of rotation, the cross-section area of the flow path of the first flow path 760A does not change. The first member 731A and the second member 731B also comprise hollows which have been bored through, in longitudinal directions, peripheral parts thereof, respectively. In the state that the flavor inhaler 700 has been assembled, the hollows in the peripheral parts of the first member 731A and the second member 731B form a part of the second flow path 760B. The shapes of cross sections, which are perpendicular to the center axis C, of the hollows in the peripheral parts are semicircular arch, as shown in the figure. Accordingly, the degree of overlap between the hollow in the peripheral part of the first member 731A and the hollow in the peripheral part of the second member 731B changes according to positional relationship between the first member 731A and the second member 731B. Thus, when the first member 731A and the second member 731B are relatively rotated about the center axis C as the axis of rotation, the cross-section area of the flow path of the second flow path 760B changes according to the degree of overlap between the hollows in the peripheral parts.

In this manner, the flow rate adjustment mechanism 730 is constructed to be able to adjust the flow rate ratio of air (and aerosol) flowing through the first flow path 760A and the second flow path 760B, by changing the cross-section area of the flow path of the second flow path 760B by relatively rotating the first member 731A and the second member 731B. A user can freely adjust the air flow rate ratio of the flow rate adjustment mechanism 730 by holding, by use of a hand, the first member 731A or the second member 731B of the flow rate adjustment mechanism 730 and turning it about the axis C of rotation. In such a case, the first member 731A and the second member 731B function as a user setting section (the user setting section 150 in Fig. 1) for setting the air flow rate ratio of the flow rate adjustment mechanism 730. Note that a variable resistor, which is not shown in the figure, is attached to the first member 731A and the second member 731B, wherein the resistance value of the variable resistor changes according to rotation of the first member 731A and the second member 731B. The control section included in the battery assembly 710 can detect the degree of opening X of the second flow path 110B based on the resistance value of the variable resistor, and use the detected degree of opening in the above explained second mode of control.

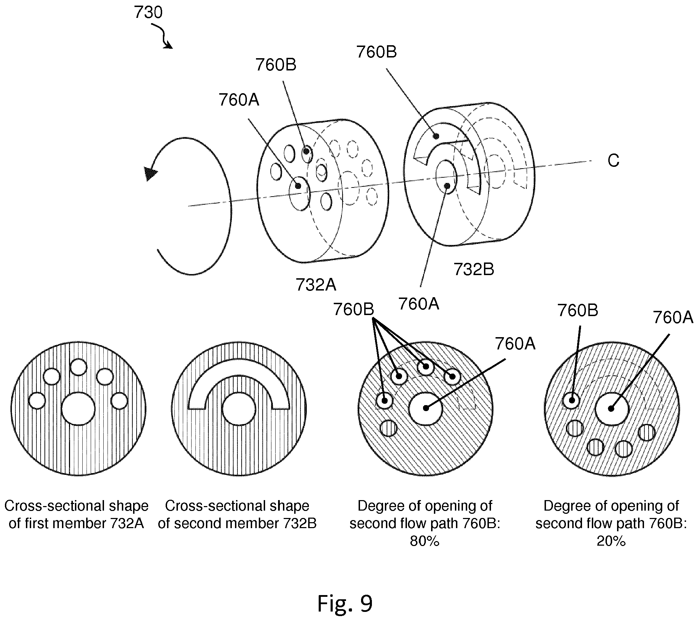

Fig. 9 is a figure showing another example of a construction of the flow rate adjustment mechanism 730 and operation thereof. The flow rate adjustment mechanism 730 comprises a first member 732A and a second member 732B. The second member 732B has a construction which is the same as that of the second member 731B in the example construction in Fig. 8. The first member 732A has a construction which is the same as that of the first member 731A in the example construction in Fig. 8, except that the shape of the hollow formed in the peripheral part is different from that of the first member 731A in the example construction in Fig. 8. Specifically, the first member 732A comprises plural small hollows (five hollows in the example in the figure) which have been bored through, in a longitudinal direction, the peripheral part of the first member 732A. The plural hollows are positioned in an approximately half circle part of the first member 732A. Accordingly, when the first member 732A and the second member 732B are relatively rotated about the center axis C as the axis of rotation, the cross-section area of the flow path of the second flow path 760B changes according to the degree of overlap between the hollows in the peripheral part of the first member 732A and the hollow in the peripheral part of the second member 732B. Thus, the air flow rate ratio of the flow rate adjustment mechanism 730 is adjustable.

Although the embodiments of the present invention have been explained in the above description, the present invention is not limited to the embodiments.

REFERENCE SIGNS LIST

| 100 | Flavor inhaler |
| 102 | Reservoir |
| 104 | Atomizing section |
| 106 | Flavor source |
| 108 | Mouthpiece member |
| 110 | Aerosol flow path |
| 110A | First flow path |
| 110B | Second flow path |
| 110C | Shared flow path |
| 112 | Flow rate adjustment mechanism |
| 114 | Battery |
| 116 | Air taking-in flow path |
| 122 | Inhaling sensor |
| 124 | Flow rate sensor |
| 130 | Control section |
| 140 | Memory |
| 150 | User setting section |

**Claims**

1. A flavor inhaler (100) comprising:

    an aerosol source;
    an atomizing section (104) for atomizing the aerosol source for generating aerosol;
    a flavor source (106) positioned downstream the atomizing section (104);
    a mouthpiece (108) positioned downstream the flavor source (106);
    an aerosol flow path (110) for guiding the aerosol generated in the atomizing section (104) to the mouthpiece (108), wherein the aerosol flow path (110) comprises a first flow path (110A) leading to the mouthpiece (108) via the flavor source (106), and a second flow path (110B) which is different from the first flow path (110A) and has a starting point connected directly or indirectly to the first flow path (110A);
a flow rate adjustment mechanism (112, 730) which can adjust a ratio between an air flow rate of the first flow path (110A) and an air flow rate of the second flow path (110B),
a control section (130) for controlling operation of at least one of the flow rate adjustment mechanism (112, 730) and the atomizing section (104),
**characterized in that** the control section (130) is constructed to control the ratio between the air flow rate of the first flow path (110A) and the air flow rate of the second flow path (110B), by controlling operation of the flow rate adjustment mechanism (112, 730) based on the amount of the aerosol generated in the atomizing section (104).

2. A flavor inhaler (100) comprising:

an aerosol source;
an atomizing section (104) for atomizing the aerosol source for generating aerosol;
a flavor source (106) positioned downstream the atomizing section (104);
a mouthpiece (108) positioned downstream the flavor source (106);
an aerosol flow path (110) for guiding the aerosol generated in the atomizing section (104) to the mouthpiece (108), wherein the aerosol flow path (110) comprises a first flow path (110A) leading to the mouthpiece (108) via the flavor source (106), and a second flow path (110B) which is different from the first flow path (110A) and has a starting point connected directly or indirectly to the first flow path (110A);
a flow rate adjustment mechanism (112, 730) which can adjust a ratio between an air flow rate of the first flow path (110A) and an air flow rate of the second flow path (110B),
a control section (130) for controlling operation of at least one of the flow rate adjustment mechanism (112, 730) and the atomizing section (104),
**characterized in that** the control section (130) is constructed to control the amount of the aerosol to be generated in the atomizing section (104) by controlling operation of the atomizing section (104) based on the ratio between the air flow rate of the first flow path (110A) and the air flow rate of the second flow path (110B).

3. A flavor inhaler (100) comprising:

an aerosol source;
an atomizing section (104) for atomizing the aerosol source for generating aerosol;
a flavor source (106) positioned downstream the atomizing section (104);
a mouthpiece (108) positioned downstream the flavor source (106);
an aerosol flow path (110) for guiding the aerosol generated in the atomizing section (104) to the mouthpiece (108), wherein the aerosol flow path (110) comprises a first flow path (110A) leading to the mouthpiece (108) via the flavor source (106), and a second flow path (110B) which is different from the first flow path (110A) and has a starting point connected directly or indirectly to the first flow path (110A);
a flow rate adjustment mechanism (112, 730) which can adjust a ratio between an air flow rate of the first flow path (110A) and an air flow rate of the second flow path (110B),
a control section (130) for controlling operation of at least one of the flow rate adjustment mechanism (112, 730) and the atomizing section (104),
**characterized in that** the control section (130) is further constructed to block communication between the atomizing section (104) and the first flow path (110A), in the case that an accumulated value of the amounts of the aerosol generated in the atomizing section (104) or an accumulated value of the amounts of the aerosol passed through the first flow path (110A) exceeds a second threshold value.

4. A flavor inhaler (100) comprising:

an aerosol source;
an atomizing section (104) for atomizing the aerosol source for generating aerosol;
a flavor source (106) positioned downstream the atomizing section (104);
a mouthpiece (108) positioned downstream the flavor source (106);
an aerosol flow path (110) for guiding the aerosol generated in the atomizing section (104) to the mouthpiece (108), wherein the aerosol flow path (110) comprises a first flow path (110A) leading to the mouthpiece (108) via the flavor source (106), and a second flow path (110B) which is different from the first flow path (110A) and has a starting point connected directly or indirectly to the first flow path (110A); and
a flow rate adjustment mechanism (112, 730) which can adjust a ratio between an air flow rate of the first flow path (110A) and an air flow rate of the second flow path (110B),
**characterized in that** the flow rate adjustment mechanism (112, 730) comprises a flow rate sensor for detecting an air flow rate of at least one of the first flow path (110A) and the second flow path (110B).

5. The flavor inhaler (100) according to one of claims 1 to 4, wherein the second flow path (110B) is a flow path which does not pass through the flavor source (106).

6. The flavor inhaler (100) according to one of claims 1 to 5, wherein the flow rate adjustment mechanism (112, 730) is positioned at least in a part on the first flow path (110A) or the second flow path (110B).

7. The flavor inhaler (100) according to claim 4 comprising a control section (130) for controlling operation of at least one of the flow rate adjustment mechanism (112, 730) and the atomizing section (104).

8. The flavor inhaler (100) according to Claim 7, wherein the control section is constructed to control the ratio between the air flow rate of the first flow path and the air flow rate of the second flow path, by controlling operation of the flow rate adjustment mechanism based on the amount of the aerosol generated in the atomizing section.

9. The flavor inhaler (100) according to Claim 8, wherein the amount of the aerosol to be generated in the atomizing section (104) per predetermined time, at the time when inhaling action starts, is determined in advance; and the control section (130) controls the air flow rate ratio, based on the predetermined amount of the aerosol to be generated per the predetermined time.

10. The flavor inhaler (100) according to Claim 8 or 9, wherein

the control section (130) is further constructed to detect change in the amount of the aerosol to be generated in the atomizing section (104) per the predetermined time, and
the control section (130) controls the air flow rate ratio based on the changed amount of the aerosol to be generated per the predetermined time.

11. The flavor inhaler (100) according to Claim 1 to 3 or 7, wherein the control section is constructed to control the amount of the aerosol to be generated in the atomizing section by controlling operation of the atomizing section based on the ratio between the air flow rate of the first flow path and the air flow rate of the second flow path.

12. The flavor inhaler (100) according to Claim 11, wherein the air flow rate ratio, at the time when inhaling action starts, is determined in advance; and the control section (130) controls the amount of the aerosol to be generated in the atomizing section (104) based on the predetermined air flow rate ratio.

13. The flavor inhaler (100) according to Claim 11 or 12, wherein

the control section (130) is further constructed to detect change in the air flow rate ratio, and the control section (130) controls the amount of the aerosol to be generated in the atomizing section (104) based on the changed air flow rate ratio.

14. The flavor inhaler (100) according to Claim 13, wherein the control section (130) determines the changed air flow rate ratio based on an operation state of the flow rate adjustment mechanism (112, 730).

15. The flavor inhaler (100) according to Claim 1 or 2, wherein the control section (130) controls at least one of the air flow rate ratio and the amount of the aerosol to be generated in the atomizing section (104), for making the amount of the aerosol passing through the first flow path (110A) to be constant.

16. The flavor inhaler (100) according to Claim 9, wherein the control section (130) controls the atomizing section (104) to change the predetermined amount of the aerosol to be generated in the atomizing section (104) per the predetermined time, in the case that an accumulated value of the amounts of the aerosol generated in the atomizing section (104) or an accumulated value of the amounts of the aerosol passed through the first flow path (110A) exceeds a first threshold value.

17. The flavor inhaler (100) according to Claim 12, wherein the control section (130) controls the flow rate adjustment mechanism (112, 730) to change the predetermined air flow rate ratio, in the case that an accumulated value of the amounts of the aerosol generated in the atomizing section (104) or an accumulated value of the amounts of the aerosol passed through the first flow path (110A) exceeds a first threshold value.

18. The flavor inhaler (100) according to one of Claims 1 to 3 or 7 to 18, wherein the control section is further constructed to block communication between the atomizing section and the first flow path or cut off supply of electric power to the atomizing section, in the case that an accumulated value of the amounts of the aerosol generated in the atomizing section or an accumulated value of the amounts of the aerosol passed through the first flow path exceeds a second threshold value.

19. The flavor inhaler (100) according to one of Claims 16 to 18, wherein the control section (130) calculates the amount of the aerosol passed through the first flow path (110A) based on the air flow rate ratio and the amount of the aerosol generated in the atomizing section (104).

20. The flavor inhaler (100) according to one of Claims 1 or 8 to 10 and 16 to 19, wherein the control section (130) calculates the amount of the aerosol generated in the atomizing section (104) based on electric energy supplied to the atomizing section (104).

21. The flavor inhaler (100) according to one of Claims 1 to 20, wherein the flow rate adjustment mechanism (112, 730) comprises a mechanism for changing at least one of a cross-section area of at least a part of the first flow path (110A) and a cross-section area of at least a part of the second flow path (110B).

22. The flavor inhaler (100) according to one of Claims 1 to 21, wherein

the flow rate adjustment mechanism (112, 730) comprises a first member (731A) and a second member (731B);
at least one of the first flow path (110A) and the second flow path (110B) is formed by the first member (731A) and the second member (731B); and
at least one of a cross-section area of at least a part of the first flow path (110A) and a cross-section area of at least a part of the second flow path (110B) is changed as a result of relative movement of the first member (731A) and the second member (731B).

23. The flavor inhaler (100) according to one of Claims 1 to 22 further comprising a battery (114) assembly comprising a battery (114).

24. The flavor inhaler (100) according to Claim 23, wherein the battery (114) assembly is attachable/detachable to/from the atomizing section (104).

25. The flavor inhaler (100) according to one of Claims 1 to 24, wherein the flow rate adjustment mechanism (112, 730) is electrically connected to the battery (114).

26. The flavor inhaler (100) according to one of Claims 1 to 25 further comprising a user setting section (150) for allowing setting of at least one of the air flow rate ratio and the amount of the aerosol to be generated in the atomizing section (104).

27. The flavor inhaler (100) according to one of Claims 1 to 26 further comprising an inhaling sensor (122) for detecting inhaling action.

28. The flavor inhaler (100) according to one of Claims 1 to 3 or 5 to 27, wherein the flow rate adjustment mechanism comprises a flow rate sensor for detecting an air flow rate of at least one of the first flow path and the second flow path.

**Patentansprüche**

1. Geschmacksinhalator (100), umfassend:

eine Aerosolquelle;
einen Zerstäubungsabschnitt (104) zum Zerstäuben der Aerosolquelle zum Erzeugen von Aerosol;
eine Geschmacksquelle (106), die stromabwärts des Zerstäubungsabschnitts (104) positioniert ist;
ein Mundstück (108), das stromabwärts der Geschmacksquelle (106) positioniert ist;
einen Aerosolströmungspfad (110) zum Führen des im Zerstäubungsabschnitt (104) erzeugten Aerosols zu dem Mundstück (108), wobei der Aerosolströmungspfad (110) einen ersten Strömungspfad (110A), der über die Geschmacksquelle (106) zu dem Mundstück (108) führt, und einen zweiten Strömungspfad (110B), der sich von dem ersten Strömungspfad (110A) unterscheidet und einen Ausgangspunkt aufweist, der direkt oder indirekt mit dem ersten Strömungspfad (110A) verbunden ist, umfasst;
einen Strömungsrateneinstellungsmechanismus (112, 730), der ein Verhältnis zwischen einer Luftströmungsrate des ersten Strömungspfads (110A) und einer Luftströmungsrate des zweiten Strömungspfads (110B) einstellen kann,
einen Steuerabschnitt (130) zum Steuern des Betriebs von mindestens einem des Strömungsrateneinstellungsmechanismus (112, 730) und des Zerstäubungsabschnitts (104),
**dadurch gekennzeichnet, dass**
der Steuerabschnitt (130) konstruiert ist, das Verhältnis zwischen der Luftströmungsrate des ersten Strömungspfads (110A) und der Luftströmungsrate des zweiten Strömungspfads (110B) zu steuern, indem Betrieb des Strömungsrateneinstellungsmechanismus (112, 730) basierend auf der Menge des im Zerstäubungsabschnitt (104) erzeugten Aerosols gesteuert wird.

2. Geschmacksinhalator (100), umfassend:

eine Aerosolquelle;
einen Zerstäubungsabschnitt (104) zum Zerstäuben der Aerosolquelle zum Erzeugen von Aerosol;
eine Geschmacksquelle (106), die stromabwärts des Zerstäubungsabschnitts (104) positioniert ist;

ein Mundstück (108), das stromabwärts der Geschmacksquelle (106) positioniert ist;

einen Aerosolströmungspfad (110) zum Führen des im Zerstäubungsabschnitt (104) erzeugten Aerosols zu dem Mundstück (108), wobei der Aerosolströmungspfad (110) einen ersten Strömungspfad (110A), der über die Geschmacksquelle (106) zu dem Mundstück (108) führt, und einen zweiten Strömungspfad (110B), der sich von dem ersten Strömungspfad (110A) unterscheidet und einen Ausgangspunkt aufweist, der direkt oder indirekt mit dem ersten Strömungspfad (110A) verbunden ist, umfasst;

einen Strömungsrateneinstellungsmechanismus (112, 730), der ein Verhältnis zwischen einer Luftströmungsrate des ersten Strömungspfads (110A) und einer Luftströmungsrate des zweiten Strömungspfads (110B) einstellen kann,

einen Steuerabschnitt (130) zum Steuern des Betriebs von mindestens einem des Strömungsrateneinstellungsmechanismus (112, 730) und des Zerstäubungsabschnitts (104),

**dadurch gekennzeichnet, dass**

der Steuerabschnitt (130) konstruiert ist, die Menge des durch den Zerstäubungsabschnitt (104) zu erzeugenden Aerosols durch Steuern des Betriebs des Zerstäubungsabschnitts (104) basierend auf dem Verhältnis zwischen der Luftströmungsrate des ersten Strömungspfads (110A) und der Luftströmungsrate des zweiten Strömungspfads (110B) zu steuern.

3.    Geschmacksinhalator (100), umfassend:

eine Aerosolquelle;

einen Zerstäubungsabschnitt (104) zum Zerstäuben der Aerosolquelle zum Erzeugen von Aerosol;

eine Geschmacksquelle (106), die stromabwärts des Zerstäubungsabschnitts (104) positioniert ist;

ein Mundstück (108), das stromabwärts der Geschmacksquelle (106) positioniert ist;

einen Aerosolströmungspfad (110) zum Führen des im Zerstäubungsabschnitt (104) erzeugten Aerosols zu dem Mundstück (108), wobei der Aerosolströmungspfad (110) einen ersten Strömungspfad (110A), der über die Geschmacksquelle (106) zu dem Mundstück (108) führt, und einen zweiten Strömungspfad (110B), der sich von dem ersten Strömungspfad (110A) unterscheidet und einen Ausgangspunkt aufweist, der direkt oder indirekt mit dem ersten Strömungspfad (110A) verbunden ist, umfasst;

einen Strömungsrateneinstellungsmechanismus (112, 730), der ein Verhältnis zwischen einer Luftströmungsrate des ersten Strömungspfads (110A) und einer Luftströmungsrate des zweiten Strömungspfads (110B) einstellen kann,

einen Steuerabschnitt (130) zum Steuern des Betriebs von mindestens einem des Strömungsrateneinstellungsmechanismus (112, 730) und des Zerstäubungsabschnitts (104),

**dadurch gekennzeichnet, dass**

der Steuerabschnitt (130) weiter konstruiert ist, Kommunikation zwischen dem Zerstäubungsabschnitt (104) und dem ersten Strömungspfad (110A) zu blockieren, falls ein akkumulierter Wert der Mengen des in dem Zerstäubungsabschnitt (104) erzeugten Aerosols oder ein akkumulierter Wert der Mengen des durch den ersten Strömungspfad (110A) gehenden Aerosols einen zweiten Schwellenwert übersteigt.

4.    Geschmacksinhalator (100), umfassend:

eine Aerosolquelle;

einen Zerstäubungsabschnitt (104) zum Zerstäuben der Aerosolquelle zum Erzeugen von Aerosol;

eine Geschmacksquelle (106), die stromabwärts des Zerstäubungsabschnitts (104) positioniert ist;

ein Mundstück (108), das stromabwärts der Geschmacksquelle (106) positioniert ist;

einen Aerosolströmungspfad (110) zum Führen des im Zerstäubungsabschnitt (104) erzeugten Aerosols zu dem Mundstück (108), wobei der Aerosolströmungspfad (110) einen ersten Strömungspfad (110A), der über die Geschmacksquelle (106) zu dem Mundstück (108) führt, und einen zweiten Strömungspfad (110B), der sich von dem ersten Strömungspfad (110A) unterscheidet und einen Ausgangspunkt aufweist, der direkt oder indirekt mit dem ersten Strömungspfad (110A) verbunden ist, umfasst; und

einen Strömungsrateneinstellungsmechanismus (112, 730), der ein Verhältnis zwischen einer Luftströmungsrate des ersten Strömungspfads (110A) und einer Luftströmungsrate des zweiten Strömungspfads (110B) einstellen kann,

**dadurch gekennzeichnet, dass**

der Strömungsrateneinstellungsmechanismus (112, 730) einen Strömungsratensensor zum Erfassen einer Luftströmungsrate von mindestens einem des ersten Strömungspfads (110A) und des zweiten Strömungspfad (110B) umfasst.

5.    Geschmacksinhalator (100) nach einem der Ansprüche 1 bis 4, wobei der zweite Strömungspfad (110B) ein Strömungspfad ist, der nicht durch die Geschmacksquelle (106) geht.

6. Geschmacksinhalator (100) nach einem der Ansprüche 1 bis 5, wobei der Strömungsrateneinstellungsmechanismus (112, 730) zumindest in einem Teil auf dem ersten Strömungspfad (110A) oder dem zweiten Strömungspfad (110B) positioniert ist.

7. Geschmacksinhalator (100) nach Anspruch 4, umfassend einen Steuerabschnitt (130) zum Steuern des Betriebs von mindestens einem des Strömungsrateneinstellungsmechanismus (112, 730) und des Zerstäubungsabschnitts (104).

8. Geschmacksinhalator (100) nach Anspruch 7, wobei der Steuerabschnitt konstruiert ist, das Verhältnis zwischen der Luftströmungsrate des ersten Strömungspfads und der Luftströmungsrate des zweiten Strömungspfads zu steuern, indem Betrieb des Strömungsrateneinstellungsmechanismus basierend auf der Menge des Aerosols gesteuert wird, die in dem Zerstäubungsabschnitt erzeugt wird.

9. Geschmacksinhalator (100) nach Anspruch 8, wobei die Menge des Aerosols, die in dem Zerstäubungsabschnitt (104) pro vorbestimmter Zeit erzeugt werden soll, zu dem Zeitpunkt, zu dem der Inhalationsvorgang startet, im Voraus bestimmt wird; und der Steuerabschnitt (130) das Luftströmungsratenverhältnis basierend auf der vorbestimmten Menge des Aerosols steuert, die pro der vorbestimmten Zeit erzeugt werden soll.

10. Geschmacksinhalator (100) nach Anspruch 8 oder 9, wobei

der Steuerabschnitt (130) weiter konstruiert ist, eine Veränderung in der Menge des Aerosols, die in dem Zerstäubungsabschnitt (104) pro der vorbestimmten Zeit erzeugt werden soll, zu erfassen, und
der Steuerabschnitt (130) das Luftströmungsratenverhältnis basierend auf der veränderten Menge des Aerosols steuert, die pro der vorbestimmten Zeit erzeugt werden soll.

11. Geschmacksinhalator (100) nach Anspruch 1 bis 3 oder 7, wobei der Steuerabschnitt konstruiert ist, die Menge des Aerosols, die in dem Zerstäubungsabschnitt erzeugt werden soll, durch Steuern des Betriebs des Zerstäubungsabschnitts basierend auf dem Verhältnis zwischen der Luftströmungsrate des ersten Strömungspfads und der Luftströmungsrate des zweiten Strömungspfads zu steuern.

12. Geschmacksinhalator (100) nach Anspruch 11, wobei das Luftströmungsratenverhältnis zu dem Zeitpunkt, zu dem der Inhalationsvorgang startet, im Voraus bestimmt wird; und der Steuerabschnitt (130) die Menge des Aerosols, die in dem Zerstäubungsabschnitt (104) erzeugt werden soll, basierend auf dem vorbestimmten Luftströmungsratenverhältnis steuert.

13. Geschmacksinhalator (100) nach Anspruch 11 oder 12, wobei

der Steuerabschnitt (130) weiter konstruiert ist, eine Veränderung in dem Luftströmungsratenverhältnis zu erfassen, und
der Steuerabschnitt (130) die Menge des Aerosols, die in dem Zerstäubungsabschnitt (104) erzeugt werden soll, basierend auf dem veränderten Luftströmungsratenverhältnis steuert.

14. Geschmacksinhalator (100) nach Anspruch 13, wobei der Steuerabschnitt (130) das veränderte Luftströmungsratenverhältnis basierend auf dem Betriebszustand des Strömungsrateneinstellungsmechanismus (112, 730) bestimmt.

15. Geschmacksinhalator (100) nach Anspruch 1 oder 2, wobei der Steuerabschnitt (130) mindestens eines von dem Luftströmungsratenverhältnis und der Menge des Aerosols, die in dem Zerstäubungsabschnitt (104) erzeugt werden soll, steuert, um die Menge des Aerosols, die durch den ersten Strömungspfad (110A) geht, konstant zu machen.

16. Geschmacksinhalator (100) nach Anspruch 9, wobei der Steuerabschnitt (130) den Zerstäubungsabschnitt (104) steuert, um die vorbestimmte Menge des Aerosols, die in dem Zerstäubungsabschnitt (104) pro der vorbestimmten Zeit erzeugt werden soll, zu verändern, falls ein akkumulierter Wert der Mengen des in dem Zerstäubungsabschnitt (104) erzeugten Aerosols oder ein akkumulierter Wert der Mengen des durch den ersten Strömungspfad (110A) gehenden Aerosols einen ersten Schwellenwert übersteigt.

17. Geschmacksinhalator (100) nach Anspruch 12, wobei der Steuerabschnitt (130) den Strömungsrateneinstellungsmechanismus (112, 730) steuert, das vorbestimmte Luftströmungsratenverhältnis zu verändern, falls ein akkumulierter Wert der Mengen des in dem Zerstäubungsabschnitt (104) erzeugten Aerosols oder ein akkumulierter Wert der Mengen des durch den ersten Strömungspfad (110A) gehenden Aerosols einen ersten Schwellenwert übersteigt.

18. Geschmacksinhalator (100) nach einem der Ansprüche 1 bis 3 oder 7 bis 18, wobei der Steuerabschnitt weiter konstruiert ist, Kommunikation zwischen dem Zerstäubungsabschnitt und dem ersten Strömungspfad zu blockieren oder Zuführung von elektrischem Strom zu dem Zerstäubungsabschnitt zu unterbrechen, falls ein akkumulierter Wert der Mengen des in dem Zerstäubungsabschnitt erzeugten Aerosols oder ein akkumulierter Wert der Mengen des durch den ersten Strömungspfad gehenden Aerosols einen zweiten Schwellenwert übersteigt.

19. Geschmacksinhalator (100) nach einem der Ansprüche 16 bis 18, wobei der Steuerabschnitt (130) die Menge des Aerosols, die durch den ersten Strömungspfad (110A) geht, basierend auf dem Luftströmungsratenverhältnis und der Menge des Aerosols, die in dem Zerstäubungsabschnitt (104) erzeugt wird, berechnet.

20. Geschmacksinhalator (100) nach einem der Ansprüche 1 oder 8 bis 10 und 16 bis 19, wobei der Steuerabschnitt (130) die Menge des Aerosols, die in dem Zerstäubungsabschnitt (104) erzeugt wird, basierend auf elektrischer Energie berechnet, die dem Zerstäubungsabschnitt (104) zugeführt wird.

21. Geschmacksinhalator (100) nach einem der Ansprüche 1 bis 20, wobei der Strömungsrateneinstellungsmechanismus (112, 730) einen Mechanismus zum Verändern mindestens einer von einer Querschnittsfläche von mindestens einem Teil des ersten Strömungspfads (110A) und einer Querschnittsfläche von mindestens einem Teil des zweiten Strömungspfads (110B) umfasst.

22. Geschmacksinhalator (100) nach einem der Ansprüche 1 bis 21, wobei

der Strömungsrateneinstellungsmechanismus (112, 730) ein erstes Element (731A) und ein zweites Element (731B) umfasst; mindestens einer von dem ersten Strömungspfad (110A) und dem zweiten Strömungspfad (110B) durch das erste Element (731A) und das zweite Element (731B) gebildet wird; und mindestens eine einer Querschnittsfläche von mindestens einem Teil des ersten Strömungspfads (110A) und einer Querschnittsfläche von mindestens einem Teil des zweiten Strömungspfads (110B) infolge einer relativen Bewegung des ersten Elements (731A) und des zweiten Elements (731B) verändert wird.

23. Geschmacksinhalator (100) nach einem der Ansprüche 1 bis 22, weiter umfassend eine Batterie- (114) Anordnung, die eine Batterie (114) umfasst.

24. Geschmacksinhalator (100) nach Anspruch 23, wobei die Batterie- (114) Anordnung an dem Zerstäubungsabschnitt (104) anbringbar/von diesem entfernbar ist.

25. Geschmacksinhalator (100) nach einem der Ansprüche 1 bis 24, wobei der Strömungsrateneinstellungsmechanismus (112, 730) elektrisch mit der Batterie (114) verbunden ist.

26. Geschmacksinhalator (100) nach einem der Ansprüche 1 bis 25, weiter umfassend einen Benutzereinstellungsabschnitt (150), um Einstellung von mindestens einem des Luftströmungsratenverhältnisses und der Menge des Aerosols, die in dem Zerstäubungsabschnitt (104) erzeugt werden soll, zu erlauben.

27. Geschmacksinhalator (100) nach einem der Ansprüche 1 bis 26, weiter umfassend einen Inhalationssensor (122) zum Erfassen eines Inhalationsvorgangs.

28. Geschmacksinhalator (100) nach einem der Ansprüche 1 bis 3 oder 5 bis 27, wobei der Strömungsrateneinstellungsmechanismus einen Strömungsratensensor zum Erfassen einer Luftströmungsrate von mindestens einem des ersten Strömungspfads und des zweiten Strömungspfads umfasst.

**Revendications**

1. Inhalateur d'arôme (100) comprenant :

une source d'aérosol ; une section d'atomisation (104) permettant d'atomiser la source d'aérosol pour générer l'aérosol ; une source d'arôme (106) positionnée en aval de la section d'atomisation (104) ; un embout buccal (108) positionné en aval de la source d'arôme (106) ; un trajet d'écoulement d'aérosol (110) permettant de guider l'aérosol généré dans la section d'atomisation (104) vers l'embout buccal (108), dans lequel le trajet d'écoulement d'aérosol (110) comprend un premier trajet d'écoulement (110A) menant à l'embout buccal (108) par le biais de la source d'arôme (106), et un second trajet d'écoulement (110B) qui est différent du premier trajet d'écoulement (110A) et présente un point de départ relié directement ou indirectement au premier trajet d'écoulement (110A) ; un mécanisme de réglage de débit (112, 730) qui peut régler un rapport entre un débit d'air du premier trajet d'écoulement (110A) et un débit d'air d'un second trajet d'écoulement (110B), une section de commande (130) permettant de commander le fonctionnement d'au moins l'un parmi le mécanisme de réglage de débit (112, 730) et la section d'atomisation (104), **caractérisé en ce que**

la section de commande (130) est construite pour commander le rapport entre le débit d'air du premier trajet d'écoulement (110A) et le débit d'air du second trajet d'écoulement (110B), en commandant le fonctionnement du mécanisme de réglage de débit (112, 730) en fonction de la quantité d'aérosol généré dans la section d'atomisation (104).

2.  Inhalateur d'arôme (100) comprenant :

    une source d'aérosol ;
    une section d'atomisation (104) permettant d'atomiser la source d'aérosol pour générer l'aérosol ;
    une source d'arôme (106) positionnée en aval de la section d'atomisation (104) ;
    un embout buccal (108) positionné en aval de la source d'arôme (106) ;
    un trajet d'écoulement d'aérosol (110) permettant de guider l'aérosol généré dans la section d'atomisation (104) vers l'embout buccal (108), dans lequel le trajet d'écoulement d'aérosol (110) comprend un premier trajet d'écoulement (110A) menant à l'embout buccal (108) par le biais de la source d'arôme (106), et un second trajet d'écoulement (110B) qui est différent du premier trajet d'écoulement (110A) et présente un point de départ relié directement ou indirectement au premier trajet d'écoulement (110A) ;
    un mécanisme de réglage de débit (112, 730) qui peut régler un rapport entre un débit d'air du premier trajet d'écoulement (110A) et un débit d'air d'un second trajet d'écoulement (110B),
    une section de commande (130) permettant de commander le fonctionnement d'au moins l'un parmi le mécanisme de réglage de débit (112, 730) et la section d'atomisation (104),
    **caractérisé en ce que**
    la section de commande (130) est construite pour commander la quantité d'aérosol à générer dans la section d'atomisation (104) en commandant le fonctionnement de la section d'atomisation (104) en fonction du rapport entre le débit d'air du premier trajet d'écoulement (110A) et le débit d'air du second trajet d'écoulement (110B).

3.  Inhalateur d'arôme (100) comprenant :

    une source d'aérosol ;
    une section d'atomisation (104) permettant d'atomiser la source d'aérosol pour générer l'aérosol ;
    une source d'arôme (106) positionnée en aval de la section d'atomisation (104) ;
    un embout buccal (108) positionné en aval de la source d'arôme (106) ;
    un trajet d'écoulement d'aérosol (110) permettant de guider l'aérosol généré dans la section d'atomisation (104) vers l'embout buccal (108), dans lequel le trajet d'écoulement d'aérosol (110) comprend un premier trajet d'écoulement (110A) menant à l'embout buccal (108) par le biais de la source d'arôme (106), et un second trajet d'écoulement (110B) qui est différent du premier trajet d'écoulement (110A) et présente un point de départ relié directement ou indirectement au premier trajet d'écoulement (110A) ;
    un mécanisme de réglage de débit (112, 730) qui peut régler un rapport entre un débit d'air du premier trajet d'écoulement (110A) et un débit d'air d'un second trajet d'écoulement (110B),
    une section de commande (130) permettant de commander le fonctionnement d'au moins l'un parmi le mécanisme de réglage de débit (112, 730) et la section d'atomisation (104),
    **caractérisé en ce que**
    la section de commande (130) est construite en outre pour bloquer la communication entre la section d'atomisation (104) et le premier trajet d'écoulement (110A), dans le cas où une valeur accumulée des quantités d'aérosol généré dans la section d'atomisation (104) ou une valeur accumulée des quantités d'aérosol passé à travers le premier trajet d'écoulement (110A) dépasse une seconde valeur seuil.

4.  Inhalateur d'arôme (100) comprenant :

    une source d'aérosol ;
    une section d'atomisation (104) permettant d'atomiser la source d'aérosol pour générer l'aérosol ;
    une source d'arôme (106) positionnée en aval de la section d'atomisation (104) ;
    un embout buccal (108) positionné en aval de la source d'arôme (106) ;
    un trajet d'écoulement d'aérosol (110) permettant de guider l'aérosol généré dans la section d'atomisation (104) vers l'embout buccal (108), dans lequel le trajet d'écoulement d'aérosol (110) comprend un premier trajet d'écoulement (110A) menant à l'embout buccal (108) par le biais de la source d'arôme (106), et un second trajet d'écoulement (110B) qui est différent du premier trajet d'écoulement (110A) et présente un point de départ relié directement ou indirectement au premier trajet d'écoulement (110A) ; et
    un mécanisme de réglage de débit (112, 730) qui peut régler un rapport entre un débit d'air du premier trajet d'écoulement (110A) et un débit d'air d'un second trajet d'écoulement (110B),
    **caractérisé en ce que**
    le mécanisme de réglage de débit (112, 730) comprend un capteur de débit pour détecter un débit d'air d'au moins l'un parmi le premier trajet d'écoulement (110A) et le second trajet d'écoulement (110B).

5. Inhalateur d'arôme (100) selon l'une des revendications 1 à 4, dans lequel le second trajet d'écoulement (110B) est un trajet d'écoulement qui ne passe pas à travers la source d'arôme (106).

6. Inhalateur d'arôme (100) selon l'une des revendications 1 à 5, dans lequel le mécanisme de réglage de débit (112, 730) est positionné au moins dans une partie située sur le premier trajet d'écoulement (110A) ou le second trajet d'écoulement (110B).

7. Inhalateur d'arôme (100) selon la revendication 4 comprenant une section de commande (130) pour commander le fonctionnement d'au moins l'un parmi le mécanisme de réglage de débit (112, 730) et la section d'atomisation (104).

8. Inhalateur d'arôme (100) selon la revendication 7, dans lequel la section de commande est construite pour commander le rapport entre le débit d'air du premier trajet d'écoulement et le débit d'air du second trajet d'écoulement, en commandant le fonctionnement du mécanisme de réglage de débit en fonction de la quantité d'aérosol généré dans la section d'atomisation.

9. Inhalateur d'arôme (100) selon la revendication 8, dans lequel la quantité d'aérosol à générer dans la section d'atomisation (104) au temps prédéterminé, au moment où l'action d'inhalation commence, est déterminée en avance ; et la section de commande (130) commande le rapport de débit d'air, en fonction de la quantité prédéterminée d'aérosol à générer au temps prédéterminé.

10. Inhalateur d'arôme (100) selon la revendication 8 ou 9, dans lequel

la section de commande (130) est construite en outre pour détecter une modification de la quantité d'aérosol à générer dans la section d'atomisation (104) au temps prédéterminé, et la section de commande (130) commande le rapport de débit d'air en fonction de la modification de la quantité d'aérosol à générer au temps prédéterminé.

11. Inhalateur d'arôme (100) selon les revendications 1 à 3 ou 7, dans lequel la section de commande est construite pour commander la quantité d'aérosol à générer dans la section d'atomisation en commandant le fonctionnement de la section d'atomisation en fonction du rapport entre le débit d'air du premier trajet d'écoulement et le débit d'air du second trajet d'écoulement.

12. Inhalateur d'arôme (100) selon la revendication 11, dans lequel le rapport de débit d'air, au moment où l'action d'inhalation commence, est déterminé en avance; et la section de commande (130) commande la quantité d'aérosol à générer dans la section d'atomisation (104) en fonction du rapport de débit d'air prédéterminé.

13. Inhalateur d'arôme (100) selon la revendication 11 ou 12, dans lequel

la section de commande (130) est construite en outre pour détecter une modification du rapport de débit d'air, et la section de commande (130) commande la quantité d'aérosol à générer dans la section d'atomisation (104) en fonction du rapport de débit d'air modifié.

14. Inhalateur d'arôme (100) selon la revendication 13, dans lequel la section de commande (130) détermine le rapport de débit d'air modifié en fonction d'un état de fonctionnement du mécanisme de réglage de débit (112, 730).

15. Inhalateur d'arôme (100) selon la revendication 1 ou 2, dans lequel la section de commande (130) commande au moins l'un parmi le rapport de débit d'air et la quantité d'aérosol à générer dans la section d'atomisation (104), de manière à maintenir la quantité d'aérosol passant à travers le premier trajet d'écoulement (110A) constante.

16. Inhalateur d'arôme (100) selon la revendication 9, dans lequel la section de commande (130) commande la section d'atomisation (104) pour modifier la quantité prédéterminée d'aérosol à générer dans la section d'atomisation (104) au temps prédéterminé, dans le cas où une valeur accumulée des quantités d'aérosol généré dans la section d'atomisation (104) ou une valeur accumulée des quantités d'aérosol passé à travers le premier trajet d'écoulement (110A) dépasse une première valeur seuil.

17. Inhalateur d'arôme (100) selon la revendication 12, dans lequel la section de commande (130) commande le mécanisme de réglage de débit (112, 730) pour modifier le rapport de débit d'air prédéterminé, dans le cas où une valeur accumulée des quantités d'aérosol généré dans la section d'atomisation (104) ou une valeur accumulée des quantités d'aérosol passé à travers le premier trajet d'écoulement (110A) dépasse une première valeur seuil.

18. Inhalateur d'arôme (100) selon l'une des revendications 1 à 3 ou 7 à 18, dans lequel la section de commande est construite en outre pour bloquer la communication entre la section d'atomisation et le premier trajet d'écoulement ou couper l'alimentation électrique de la section d'atomisation, dans le cas où une valeur accumulée des quantités d'aérosol généré dans la section d'atomisation ou une valeur accumulée des quantités d'aérosol passé à travers le premier trajet d'écoulement dépasse une seconde valeur seuil.

19. Inhalateur d'arôme (100) selon l'une des revendications 16 à 18, dans lequel la section de commande (130) calcule la quantité d'aérosol passé à travers le premier trajet d'écoulement (110A) en fonction du rapport de débit d'air et la quantité d'aérosol généré dans la section d'atomisation (104).

20. Inhalateur d'arôme (100) selon l'une des revendications 1 ou 8 à 10 et 16 à 19, dans lequel la section de commande (130) calcule la quantité d'aérosol généré dans la section d'atomisation (104) en fonction de l'énergie électrique fournie à la section d'atomisation (104).

21. Inhalateur d'arôme (100) selon l'une des revendications 1 à 20, dans lequel le mécanisme de réglage de débit (112, 730) comprend un mécanisme permettant de modifier au moins l'une parmi une aire de section transversale d'au moins une partie du premier trajet d'écoulement (110A) et une aire de section transversale d'au moins une partie du second trajet d'écoulement (110B).

22. Inhalateur d'arôme (100) selon l'une des revendications 1 à 21, dans lequel

le mécanisme de réglage de débit (112, 730) comprend un premier élément (731A) et un second élément (731B) ;
au moins l'un parmi le premier trajet d'écoulement (110A) et le second trajet d'écoulement (110B) est formé par le premier élément (731A) et le second élément (731B) ; et
au moins l'une parmi une aire de section transversale d'au moins une partie du premier trajet d'écoulement (110A) et une aire de section transversale d'au moins une partie du second trajet d'écoulement (110B) est modifiée en raison d'un mouvement relatif du premier élément (731A) et du second élément (731B).

23. Inhalateur d'arôme (100) selon l'une des revendications 1 à 22 comprenant en outre un ensemble formant batterie (114) comprenant une batterie (114).

24. Inhalateur d'arôme (100) selon la revendication 23, dans lequel l'ensemble formant batterie (114) peut être fixé/détaché à/de la section d'atomisation (104).

25. Inhalateur d'arôme (100) selon l'une des revendications 1 à 24, dans lequel le mécanisme de réglage de débit (112, 730) est raccordé électriquement à la batterie (114).

26. Inhalateur d'arôme (100) selon l'une des revendications 1 à 25 comprenant en outre une section de réglage de l'utilisateur (150) permettant de régler au moins l'un parmi le rapport de débit d'air et la quantité d'aérosol à générer dans la section d'atomisation (104).

27. Inhalateur d'arôme (100) selon l'une des revendications 1 à 26 comprenant en outre un capteur d'inhalation (122) pour détecter l'action d'inhalation.

28. Inhalateur d'arôme (100) selon l'une des revendications 1 à 3 ou 5 à 27, dans lequel le mécanisme de réglage de débit comprend un capteur de débit pour détecter un débit d'air d'au moins l'un parmi le premier trajet d'écoulement et le second trajet d'écoulement.

Fig. 1

EP 3 491 945 B1

```
┌─────────────────────────────────────┐
│ Determining the amount of aerosol to be │ ──S202
│ generated in the atomizing section      │
└─────────────────────────────────────┘
                  │
                  ▼
┌─────────────────────────────────────┐
│ Determining the amount of aerosol       │ ──S204
│ supplied to the flavor source           │
└─────────────────────────────────────┘
                  │
                  ▼
┌─────────────────────────────────────┐
│ Determining an air flow rate ratio of the │
│ flow rate adjustment mechanism, based on  │ ──S206
│ the amount of aerosol generated in the    │
│ atomizing section and the amount of       │
│ aerosol supplied to the flavor source     │
└─────────────────────────────────────┘
                  │
                  ▼
┌─────────────────────────────────────┐
│ Controlling the flow rate adjustment     │ ──S208
│ mechanism                                │
└─────────────────────────────────────┘
```

Fig. 2

```
┌─────────────────────────────────┐
│ Calculating an air flow rate ratio of the flow │ ⌇S302
│      rate adjustment mechanism      │
└─────────────────────────────────┘
                │
                ▼
┌─────────────────────────────────┐
│   Determining the amount of aerosol   │ ⌇S304
│       supplied to the flavor source      │
└─────────────────────────────────┘
                │
                ▼
┌─────────────────────────────────┐
│  Determining the amount of aerosol to be │
│ generated in the atomizing section, based │ ⌇S306
│   on the air flow rate ratio of the flow rate │
│  adjustment mechanism and the amount of │
│    aerosol supplied to the flavor source   │
└─────────────────────────────────┘
                │
                ▼
┌─────────────────────────────────┐
│       Controlling the atomizing section    │ ⌇S308
└─────────────────────────────────┘
```

Fig. 3

Fig. 4

Fig. 5

Fig. 6

Non-mouthpiece side ⟵————⟶ Mouthpiece side

A

700

General view

760

760A 760B 760A 760B

Exploded view

710    720    730    740    750

Fig. 7

EP 3 491 945 B1

Cross-sectional shape of first member 731A

Cross-sectional shape of second member 731B

Degree of opening of second flow path 760B: 75%

Degree of opening of second flow path 760B: 25%

Fig. 8

EP 3 491 945 B1

EP 3 491 945 B1

730

760B

760B

760A

732A

760B  760A

732B

760A

C

| Cross-sectional shape of first member 732A | Cross-sectional shape of second member 732B | Degree of opening of second flow path 760B: 80% | Degree of opening of second flow path 760B: 20% |

760B  760A

760B  760A

Fig. 9

**EP 3 491 945 B1**

**Patent documents cited in the description**

- WO 2015179388 A1

- US 2015258288 A1